(19) Europäisches Patentamt
European Patent Office
Office européen des brevets

(11) EP 1 808 078 A1

(12) EUROPEAN PATENT APPLICATION
published in accordance with Art. 158(3) EPC

(43) Date of publication:
18.07.2007 Bulletin 2007/29

(21) Application number: 05790121.7

(22) Date of filing: 28.09.2005

(51) Int Cl.:
*A23F 5/18* (2006.01) *A23F 5/04* (2006.01)
*A23L 1/30* (2006.01) *A61P 3/02* (2006.01)
*A61P 3/06* (2006.01) *A61P 3/10* (2006.01)

(86) International application number:
PCT/JP2005/018394

(87) International publication number:
WO 2006/035998 (06.04.2006 Gazette 2006/14)

(84) Designated Contracting States:
DE FR GB IT

(30) Priority: 30.09.2004 JP 2004289202
22.02.2005 JP 2005046258
25.03.2005 JP 2005088882

(71) Applicant: Tama-Tlo Corporation
Hachioji-shi,
Tokyo 192-0083 (JP)

(72) Inventors:
- OKA, Kitaro
Tokyo 146-0094 (JP)
- KAGAMI, Keisuke
Hachioji-shi, Tokyo 192-0045 (JP)
- HARA, Takafumi
HINO-SHI, TOKYO 191-0043 (JP)
- SUGIMOTO, SAHO
KAWASAKI-SHI, KANAGAWA 214-0008 (JP)

(74) Representative: Forstmeyer, Dietmar
BOETERS & LIECK
Oberanger 32
80331 München (DE)

# (54) MODIFIED COFFEE, METHOD OF ROASTING COFFEE BEAN, COFFEE-LIKE SUPPLEMENT AND AUXILIARY FOOD

(57) A modified coffee containing 3 mg or more of a nicotinic acid compound and 10 mg or more of a Maillard reaction product per 10 g of roasted coffee beans. A coffee-like supplement containing at least one nicotinic acid compound, at least one Maillard reaction product, and/or at least one metabolite of the Maillard reaction product.

EP 1 808 078 A1

## Description

TECHNICAL FIELD

The present invention relates to modified coffee with increased contents of health-promoting components and a roasting method for obtaining the modified coffee. Also, the present invention relates to supplements and supplemented foods containing roasted coffee components that are health-promoting components, more preferably coffee-like supplements and coffee-like supplemented foods.

BACKGROUND ART

Coffee products have unique tastes, which reduce stresses that occur in modern society, give a relaxed feeling, and the like, so people love them as favorite beverages. On the other hand, there has been a concern that taking large amounts of conventional coffee products increases the risk of cardiovascular diseases (see, for example, J. Nutr. 134: 2381-2386 (2004)).

For avoiding the above concern and the stimulation by caffeine or for other reasons, decaffeinated coffee (hereinafter, simply referred to as "decaf") has been widely consumed. However, decaf products have the defects in poor tastes since a delicious taste component and a flavor component are lost during any caffeine extracting step, such as a water extraction method.

Meanwhile, a Maillard reaction product that is known to be contained in small amounts in roasted beans as an aroma and taste component is a brown substance called melanoidin that is formed by a reaction of amino acid with a reducing sugar such as glucose or fructose during roasting. An example of the Maillard reaction product is a product obtained by adding water to glucose and alanine to dissolve them and heating the solution to allow the reaction.

Similarly to the Maillard reaction product, roasted coffee is known to contain vitamin $B_3$ (nicotinic acid and nicotinamide) and be effective to a vitamin $B_3$ deficiency disease (pellagra), so that the content of vitamin $B_3$ in coffee has been studied (see, for example, Agric. Biol. Chem. 49(12), 3467-3471, 1985, and Nutritional and Toxicological Consequences of Food Processing, 49-59, M. Friedman, Plenum Press, New York, 1991 edition, Eur. J. Med. Chem. 15: pp157-163 (1980)).

A physiological action of vitamin $B_3$ is one as coenzyme NAD, which activates a metabolic pathway for producing energy. Nicotinic acid as the vitamin is commercially available as a general drug, which denotes an action of stimulating metabolism of glucides and lipids. On the other hand, nicotinic acid approved as a drug for medical application is a therapeutic agent for hyperlipidemia and its pharmacological characteristic is effective for an improvement of lipid metabolism, in particular, increasing blood HDL-C level and for prevention and therapy of hyperlipidemia and diabetes (see, for example, Arch. Int. Med. 2004:164, 697-705). Nicotinic acid has the most potent HDL-C-increasing action among the existing drugs.

Nicotinic acid is known to show, as a side effect, a phenomenon that continued administration of nicotinic acid causes an abrupt increase of once reduced blood lipid level by a rebound (hereinafter, simply referred to as a "rebound phenomenon"). Fig. 12 is a graph showing the rebound phenomenon of nicotinic acid described later in the section of EXAMPLE.

As will be apparent from Fig. 12, in a system in which nicotinic acid was administered, once reduced blood lipid level abruptly increased after 1 hour with dose of 5 mg/kg, after 2 hours with 10 to 20 mg/kg, and after 4 hours with 50 mg/kg by rebound and exceeded over the blood level without administration.

In addition, with respect to human, the same results are conformed.

To alleviate the side effect of nicotinic acid as a drug for medical application, Acipimox (trade name, manufactured by Pharmacia in Italy/now, Pfizer) has been developed taking nicotinic acid as a model (see, for example, Clin. Pharmacol. Ther. (1980) Vol. 28, Number 6, 790-795) and used mainly in Europe in the therapy of hyperlipidemia (in Japan and U. S. A., it remains to be approved).

In the drug therapy of patients showing high levels of total blood cholesterol among those patients suffering from the above hyperlipidemia, it is effective and general to use statin-based drugs. However, the statin-based drugs have a defect that they have almost no HDL-C-increasing effect. Recently, there have been a wave of reports on an enhanced increase in HDL-C when the statin-based drug is used in combination with nicotinic acid as compared when each of them is used singly (see, for example, Am. Heart J. 2002:143, 514-8, Am. J. Cardiol. 2004:93, 307-12, and Am. J. Cardiol. 2004:94, 306-11). In particular, a method of using a small amount of nicotinic acid in combination with a statin-based drug (Am. Heart J. 2002:143, 514-8) is effective since it enables safe and long-term administration and causes less rebound phenomenon.

That is, it is considered that suppression of total blood cholesterol and simultaneous control of HDL-C level at a high level will prevent diabetes and arteriosclerosis and reduce the risk rate of developing lethal vascular diseases and brain infarction caused by the lifestyle-related diseases. Table 1 shows examples of drugs that have a cholesterol-reducing action and drugs that have an HDL-C-increasing action.

Table 1. Cholesterol-reducing drug and HDL-C-increasing drug

| I. Cholesterol-reducing drug | II. HDL-C-increasing drug |
|---|---|
| Statin-based drug (biosynthesis inhibitor) | Nicotinic acid (HM74 ligand)<br>Acipimox (HM74 ligand) |

On the other hand, most recently, it has been epidemiologically demonstrated that coffee has an effect of preventing development of type two diabetes (see, for example, Lancet 2002, 360:1477-1478, Lancet, 2003, 361: 702-704, Annals of Internal Medicine, 2004; 140:1-8, Journal of Internal Medicine, 2004; 255:89-95, Annals of Internal Medicine, 2004;140:1-8, Journal of Internal Medicine, 2004;255:89-95, and JAMA., 2004;291:1213-1219). However, the mechanism in which the effect is exhibited is quite unclear (see, in particular Annals of Internal Medicine, 2004; 140: 1-8, Journal of Internal Medicine, 2004;255:89-95, and JAMA., 2004;291:1213-1219).

Although there have been no report on prevention of hyperlipidemia, obesity, or diabetes by nicotinic acid or nicotinamide contained in coffee, components similar to nicotinic acid and nicotinamide known as the drug are included in the compounds contained in roasted coffee.

However, even in the case of roasted coffee, those products other than French and Italian cannot be said to contain sufficient nicotinic acid (see, for example, Anal. Sci., 20, 325-328 (2004)).

Further, conventional commercially available roasted coffee products, known to contain a minute amount of a Maillard reaction product as described above, contain caffeine, which is a main component of flavor and taste of original coffee, therefore they should not be taken in large amounts. No products enriched with both the nicotinic acid and Maillard reaction products in increased content ratios that are suitable for preventing diabetes have been obtained. Much less, there has been no safe modified coffee, supplement, or supplemented food that suppresses the risk of cardiovascular disease which the conventional coffee products have and has a preventive effect for diabetes.

DISCLOSURE OF INVENTION

A subject of the present invention is to provide a coffee product that has a relatively reduced amount of caffeine and contains nicotinic acid and a Maillard reaction product that are health-promoting components in increased ratios.

More particularly, an subject of the present invention is to provide modified coffee that prevents diabetes and suppresses the risk of cardiovascular diseases and a roasting method for obtaining thereof.

Further, a subject of the present invention is to provide a coffee-like supplement and supplemented food that has a relatively reduced amount of caffeine and contains nicotinic acid and a Maillard reaction product that are health-promoting components in increased ratios. More particularly, a subject of the present invention is to provide a supplement and a supplemented food that prevent diabetes and suppresses the risk of cardiovascular diseases.

Moreover, a subject of the present invention is to provide a supplement that reduces the amount of caffeine to a relatively low level by being blended in any coffee (in particular, any decaf product) as a blend component thereby to supplement or improve a delicious taste and flavor of blended coffee.

To prepare products containing much nicotinic acid from green coffee beans, high temperatures reaching 220˚C are preferable. However, since the Maillard reaction product is reported to have a high volatility, a relatively low temperature of below 200˚C would be preferable though no fixed idea was present. Accordingly, the inventors of the present invention made extensive studies on roasting time and temperature and as a result have found that the contents of the nicotinic acid and the Maillard reaction product can be increased simultaneously and realized a modified coffee and a supplement suitable for preventing diabetes and the like each containing both compounds in preferable ratios.

Further, as a result of extensive studies, the inventors of the present invention have found that when the Maillard reaction products contained in roasted coffee beans are incorporated into the body, they are converted into components that reduce blood lipid level by liver metabolism and the metabolites thereof prevent rebound phenomenon which nicotinic acids have and reduce blood lipid level. The present invention has been accomplished in accordance with those findings.

According to the present invention, the following means are provided.

(1) A modified coffee containing 3 mg or more of a nicotinic acid compound and 10 mg or more of a Maillard reaction product per 10 g of roasted coffee beans.

(2) The modified coffee according to the above item (1), containing 30 mg or more of the Maillard reaction product.

(3) The modified coffee, in which at least one of the nicotinic acid compound and at least one of the Maillard reaction product have respective contents in increased ratios as compared with a standard amount of caffeine from roasted coffee beans.

(4) The modified coffee according to any one of the above items (1) to (3), in which the nicotinic acid is represented by the following general formula A,

Formula A

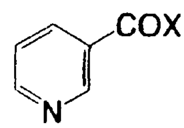

wherein X represents a hydroxyl group, an amino group or a methoxy group.

(5) The modified coffee according to any one of the above items (1) to (4), in which the Maillard reaction product contains at least one compound represented by the following formula B1, formula B2 or formula B3,

Formula B1

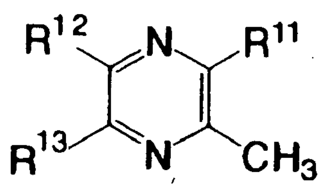

Formula B2

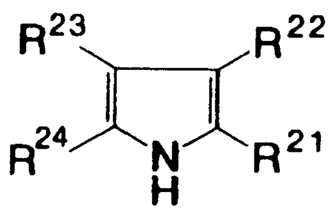

Formula B3

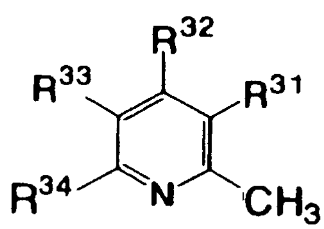

wherein, $R^{11}$ to $R^{13}$, $R^{21}$ to $R^{24}$, and $R^{31}$ to $R^{34}$ each represent a hydrogen atom or a methyl group, provided that $R^{21}$ may be an aldehyde group.

(6) The modified coffee according to any one of the above items (1) to (5), in which a coffee extract solution obtained from the roasted coffee beans is dried.

(7) A method of roasting coffee beans, comprising roasting green coffee beans at 200 to 230˚C for 15 to 25 minutes.
(8) A method of roasting coffee beans, comprising roasting green coffee beans at 180 to 200˚C for 25 to 40 minutes.
(9) A method of roasting coffee beans, comprising roasting green coffee beans at a temperature of Y for a time of X satisfying the relationship represented by the following Formula 1:

Formula 1

$$X=240 - Y$$

wherein Y is 180 to 220˚C and X is 20 to 60 minutes.
(10) The method of roasting coffee beans according to the above item (9), in which Y is 180 to 200˚C and X is 40 to 60 minutes.
(11) The method of roasting according to any one of the above items (7) to (10), in which the green coffee beans contain 300 mg or more of trigonelline in 100 g of the green beans.
(12) The modified coffee obtained by the method of roasting according to any one of the above items (7) to (11).
(13) A modified coffee, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

(14) A modified coffee, comprising at least two kinds of the following roasted coffee beans (d) to (f):

(d) a coffee bean obtained by roasting a green coffee bean as a raw material at 180 to 220˚C for 1 to 6 minutes;
(e) a coffee bean obtained by roasting a green coffee bean as a raw material at 190 to 225˚C for 7 to 14 minutes; and
(f) a coffee bean obtained by roasting a green coffee bean as a raw material at 200 to 230˚C for 15 to 30 minutes.

(15) A modified coffee, comprising roasted coffee beans in each of which contents of chlorogenic acid, at least one nicotinic acid compound, and at least one Maillard reaction product are increased with respect to a certain amount of caffeine in the roasted coffee beans.
(16) The modified coffee according to item (15), wherein the mixture further comprises a roasted coffee bean including chlorogenic lactone in an increased amount with respect to a certain amount of caffeine in the roasted coffee bean.
(17) A modified coffee, comprising 30 mg or more of chlorogenic acid, 3 mg or more of a nicotinic acid compound, and 10 mg or more of a Maillard reaction product per 150 ml of a coffee extract.
(18) The modified coffee according to item (17), further comprising 1 mg or more of chlorogenic lactone.
(19) A coffee-like supplement containing at least one nicotinic acid compound, at least one Maillard reaction product, and/or at least one metabolite of the Maillard reaction product.
(20) The supplement according to the above item (19), in which the nicotinic acid compound is represented by the following formula A,

Formula A

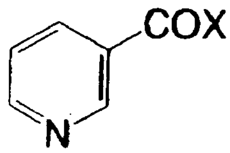

wherein X represents a hydroxyl group, an amino group or a methoxy group:
(21) The supplement according to any one of the above items (19) to (20), in which the Maillard reaction product contains at least one compound represented by the following formula B1, formula B2, or formula B3,

Formula B1

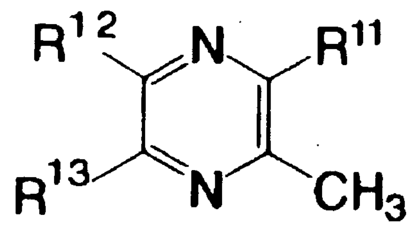

Formula B2

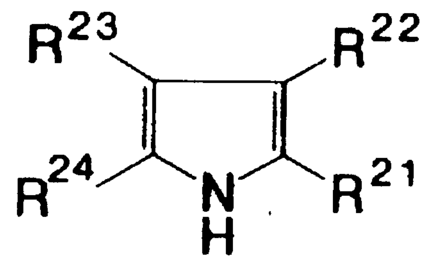

Formula B3

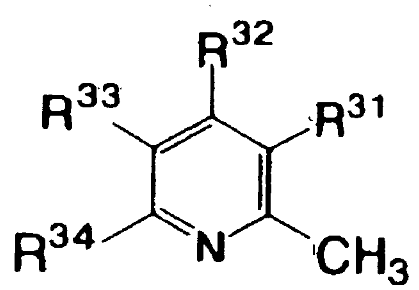

wherein $R^{11}$ to $R^{13}$, $R^{21}$ to $R^{24}$, and $R^{31}$ to $R^{34}$ each represent a hydrogen atom or a methyl group, provided that $R^{21}$ may be an aldehyde group.

(22) The supplement according to any one of the above items (19) to (21), in which the Maillard reaction product contains at least one compound represented by the following formula C1, formula C2, or formula C3,

Formula C1

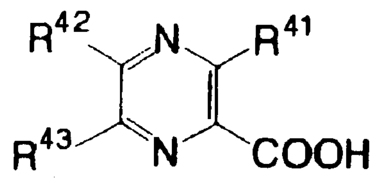

Formula C2

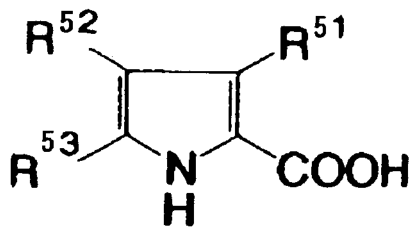

Formula C3

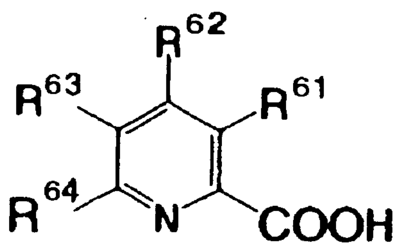

wherein $R^{41}$ to $R^{43}$, $R^{51}$ to $R^{53}$, and $R^{61}$ to $R^{64}$ each represent a hydrogen atom or a methyl group.

(23) The supplement according to any one of the above items (19) to (22), in which the supplement is prepared from roasted coffee.

(24) The supplement according to any one of the above items (19) to (23), in which the nicotinic acid compound, the Maillard reaction product and/or the metabolite of the Maillard reaction product have respective contents in increased ratios as compared with a standard amount of caffeine derived from the roasted coffee beans.

(25) The supplement according to any one of the above items (19) to (24), in which the supplement contains 3 mg or more of the nicotinic acid compound and a total 30 mg or more of the Maillard reaction product and the metabolite of the Maillard reaction product in a total 10 g of components obtained from the roasted coffee beans.

(26) The supplement according to any one of the above items (19) to (25), in which the supplement contains 3 mg or more of the nicotinic acid and at least 30 mg of the Maillard reaction product in a total 10 g of components obtained from the roasted coffee beans.

(27) The supplement according to any one of the above items (19) to (26), in which the nicotinic acid compound and the Maillard reaction compound are obtained from roasted coffee beans obtained by roasting green coffee

beans at 200 to 230°C for 15 to 25 minutes.

(28) A coffee-like supplement, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

(29) A coffee-like supplement, comprising at least two kinds of the following roasted coffee beans (d) to (f):

(d) a coffee bean obtained by roasting a green coffee bean as a raw material at 180 to 220°C for 1 to 6 minutes;
(e) a coffee bean obtained by roasting a green coffee bean as a raw material at 190 to 225°C for 7 to 14 minutes; and
(f) a coffee bean obtained by roasting a green coffee bean as a raw material at 200 to 230°C for 15 to 30 minutes.

(30) A coffee-like supplement, comprising a mixture of roasted coffee beans in each of which contents of chlorogenic acid, at least one nicotinic acid compound, and at least one Maillard reaction product are increased with respect to a certain amount of caffeine in the roasted coffee beans.

(31) The coffee-like supplement according to item (30), wherein the mixture further comprises a roasted coffee bean including chlorogenic lactone in an increased amount with respect to a certain amount of caffeine in the roasted coffee bean.

(32) A coffee-like supplement, comprising 30 mg or more of chlorogenic acid, 3 mg or more of a nicotinic acid compound, and 10 mg or more of a Maillard reaction product per 10 g in total of components obtained from a roasted coffee bean.

(33) The coffee-like supplement according to item 32, further comprising 1 mg or more of chlorogenic lactone.

(34) A coffee-like supplemented food containing at least one nicotinic acid compound, at least one Maillard reaction product and/or at least one metabolite product of the Maillard reaction product.

(35) The supplemented food according to the above item (34), in which the nicotinic acid is represented by the following formula A,

Formula A

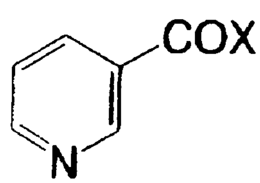

wherein X represents a hydroxyl group, an amino group, or a methoxy group.

(36) The supplemented food according to any one of the above items (34) and (35), in which the Maillard reaction product contains at least one compound represented by the following formula B1, formula B2, or formula B3,

Formula B1

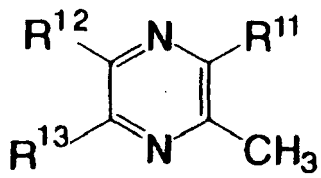

Formula B2

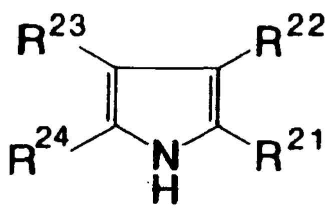

Formula B3

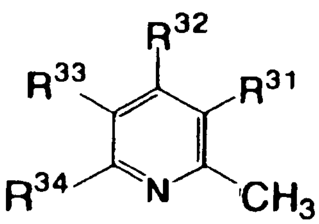

wherein $R^{11}$ to $R^{13}$, $R^{21}$ to $R^{24}$, and $R^{31}$ to $R^{34}$ each represent a hydrogen atom or a methyl group, provided that $R^{21}$ may be an aldehyde group.

(37) The supplemented food according to any one of the above items (34) to (36), in which the Maillard reaction product contains at least one compound represented by the following formula C1, formula C2, or formula C3,

Formula C1

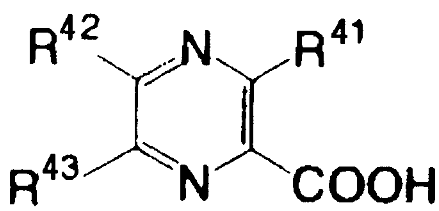

Formula C2

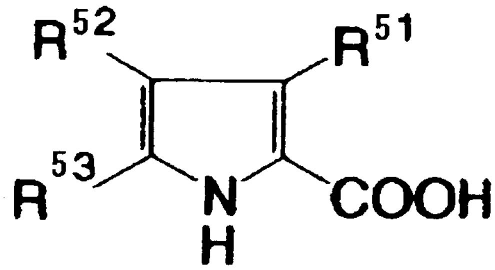

Formula C3

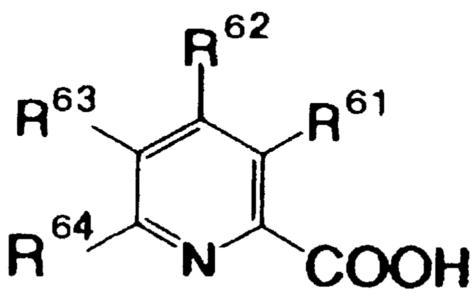

wherein $R^{41}$ to $R^{43}$, $R^{51}$ to $R^{53}$, $R^{61}$ to $R^{64}$ each represent a hydrogen atom or a methyl group.

(38) The supplemented food according to any one of the above items (34) to (37), in which the supplemented food is prepared from roasted coffee.

(39) The supplemented food according to any one of the above items (34) to (38), in which the nicotinic acid compound, the Maillard reaction product and/or the metabolite of the Maillard reaction product have respective contents in increased ratios as compared with a standard amount of caffeine derived from the roasted coffee beans.

(40) The supplemented food according to any one of the above items (34) to (39), in which the supplemented food contains 3 mg or more of the nicotinic acid compound and a total 30 mg or more of the Maillard reaction product and the metabolite of the Maillard reaction product in a total 10 g of components obtained from the roasted coffee beans.

(41) The supplemented food according to any one of the above items (34) to (40), in which the supplemented food contains 3 mg or more of the nicotinic acid and 30 mg or more of the Maillard reaction product in a total 10 g of components obtained from the roasted coffee beans.

(42) The supplemented food according to any one of the above items (34) to (41), in which the nicotinic acid compound and the Maillard reaction compound are obtained from roasted coffee beans obtained by roasting green coffee beans at 200 to 230°C for 15 to 25 minutes.

(43) A coffee-like supplemented food, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

(44) A coffee-like supplemented food, comprising at least two kinds of the following roasted coffee beans (d) to (f):

(d) a coffee bean obtained by roasting a green coffee bean as a raw material at 180 to 220°C for 1 to 6 minutes;
(e) a coffee bean obtained by roasting a green coffee bean as a raw material at 190 to 225°C for 7 to 14 minutes; and
(f) a coffee bean obtained by roasting a green coffee bean as a raw material at 200 to 230°C for 15 to 30 minutes.

(45) A coffee-like supplemented food, comprising roasted coffee beans in each of which contents of chlorogenic acid, at least one nicotinic acid compound, and at least one Maillard reaction product are increased with respect to a certain amount of caffeine in the roasted coffee beans.
(46) The coffee-like supplemented food according to item (45), wherein the mixture further comprises a roasted coffee bean including chlorogenic lactone in an increased amount with respect to a certain amount of caffeine in the roasted coffee bean.
(47) A coffee-like supplemented food, comprising 30 mg or more of chlorogenic acid, 3 mg or more of a nicotinic acid compound, and 10 mg or more of a Maillard reaction product per 10 g in total of components obtained from a roasted coffee bean.
(48) The coffee-like supplemented food according to item (47), further comprising 1 mg or more of chlorogenic lactone.

The modified coffee of the present invention is a coffee obtained from roasted coffee beans that contain nicotinic acid and a Maillard reaction product abundantly. Preferably, it contains 3 mg or more (more preferably 4 mg or more) of nicotinic acid and 10 mg or more (more preferably 30 mg or more, still more preferably 50 mg or more) of the Maillard reaction product.
The supplement and supplemented food of the present invention are a supplement and a supplemented food each contain nicotinic acid, a Maillard reaction product, and/or a metabolite of the Maillard reaction product, preferably containing 3 mg or more (more preferably 4 mg or more) of nicotinic acid and 30 mg or more (more preferably 50 mg or more) of the Maillard reaction product per 10 g.
In the present invention, the term "coffee-like" means not only supplements and supplemented foods prepared from coffee beans but also includes those products that have flavor and taste of coffee by blending the respective components mentioned above (including synthetic products).
According to the roasting method of the present invention, roasted coffee abundantly containing nicotinic acid and the Maillard reaction product can be obtained.
The modified coffee obtained by the roasting method of the present invention prevents the rebound phenomenon of nicotinic acid to achieve a sustaining blood lipid level-reducing effect and have also an HDL-C-increasing effect, since the slow-acting blood lipid level-reducing effect caused by the metabolites of the Maillard reaction products in liver complements the fast-acting blood lipid level-reducing effect caused by nicotinic acid. From this, by making the best of the lifestyle of drinking coffee, the development of the type 2 diabetes and arteriosclerosis can be prevented for a long period of life.
Further, the modified coffee of the present invention in daily life, when people drink it, decreases the influence of excessive intake of caffeine and also decreases the risk of a blood pressure increase that is said to be due to a large amount of conventional coffee, so that the risk of cardiovascular diseases and heart diseases relatively decrease.
For the type 2 diabetes preventing effect, the modified coffee of the present invention can be formulated such that one cup or a few cups of the modified coffee of the present invention corresponds to 7 to 10 cups of the conventional coffee.
The supplement and supplemented food of the present invention (hereinafter, referred to as "supplement" collectively) contain nicotinic acid, the Maillard reaction products, and/or the metabolites of the Maillard reaction products abundantly.
The supplement of the present invention can prevent the development of the type 2 diabetes and arteriosclerosis to alleviate the risk of the development of cardiovascular diseases by making the supplement composed of components obtained from particular roasted coffee beans to be contained in food that is used regularly and daily, for example, most directly, making best of the lifestyle of drinking coffee.
The supplement of the present invention can be used to prepare a blended coffee that can decrease the influence of excessive intake of caffeine and the risk of a blood pressure increase that is said to be promoted by drinking a large amount of the conventional coffee and, on the other hand, can decrease the risk of cardiovascular diseases and heart diseases relatively when taken in a daily life as blended in a predetermined amount in coffee as a blend component. In addition, the supplement of the present invention can be used as blend component that has the taste unique to the Maillard reaction product and allows people to enjoy aroma and taste of original coffee when blended in decaf products.
When the supplement of the present invention is blended in coffee, the type 2 diabetes-preventing effect of the coffee can be made such that one cup or a few cups of the modified coffee of the present invention corresponds to

7 to 10 cups of conventional coffee.

The supplement of the present invention prevents the rebound phenomenon of nicotinic acid to achieve a sustaining blood lipid level-reducing effect and have also HDL-C-increasing effect since the slow-acting blood lipid level-reducing effect by the metabolites of the Maillard reaction products in liver complements the fast-acting blood lipid level-reducing effect by nicotinic acid.

Other and further features and advantages of the invention will appear more fully from the following description, appropriately referring to the accompanying drawings.

## BRIEF DESCRIPTION OF DRAWINGS

In the accompanying drawings:

Fig. 1 is a schematic diagram for explaining the action of an HM74 ligand and an intracellular signal transduction pathway in an adipose cell;
Fig. 2 is a diagram showing a chromatogram of nicotinic acid contained in roasted coffee beans;
Fig. 3 is a graph showing results of determination of a relationship between the amount of nicotinic acid produced and the roasting time for every 10 minutes in a range of 180 to 220˚C;
Fig. 4-1 is a diagram showing NMR spectrum of an aqueous solution of coffee used in quantitative assay of a Maillard reaction product;
Fig. 4-2 is a diagram showing NMR spectrum of a chloroform solution of coffee used in quantitative assay of a Maillard reaction product;
Fig. 4-3 is a partially enlarged view of the spectrum of Fig. 4-2 within the range of 2.0 to 2.5 ppm;
Fig. 4-4 is a diagram showing NMR spectrum of an aqueous solution of coffee used in quantitative assay of a Maillard reaction product;
Fig. 4-5 is a partially enlarged view of the spectrum of a Maillard reaction product in the vicinity of 9 ppm;
Fig. 4-6 (a) is a partially enlarged view of the NMR spectrum shown in Fig. 4-4 within the range of 2.2 to 3.5 ppm;
Fig. 4-6 (b) shows NMR spectrum (2.2 to 3.5 ppm) of a solution obtained by extraction of commercially available roasted coffee originated from Brazilian coffee beans with hot water;
Fig. 5-1 is a diagram showing time course of the amount of a Maillard reaction product at a roasting temperature of 200˚C;
Fig. 5-2 is a diagram showing time course of the amounts of caffeine, trigonelline, chlorogenic acid, chlorogenic lactone, nicotinic acid, Maillard reaction products, and sucrose at a roasting temperature of 200˚C;
Fig. 6 is a diagram showing a chromatogram of metabolites in urine of a rat administered with monomethylpyrazine;
Fig. 7 is a diagram showing a chromatogram of metabolites in urine of a rat administered with pyrrole-2-aldehyde;
Fig. 8 is a diagram showing a chromatogram of metabolites in urine of a rat administered with 2,5-dimethylpyrazine;
Fig. 9 is a diagram showing a blood level-time curve of 2,5-dimethylpyrazine in blood of a rat administered with 2,5-dimethypyrazine and its liver metabolite, 5-methylpyrazinecarboxylic acid;
Fig. 10 is a diagram showing blood free fatty acid level-time curve of a rat administered with 5-methylpyrazinecarboxylic acid;
Fig. 11 (a) and (b) are diagrams showing effects of nicotinic acid and pyrazinecarboxylic acids on lipid metabolism;
Fig. 12 is a graph showing a dose dependent rebound phenomenon with nicotinic acid; and
Fig. 13 is a graph showing time course of blood free fatty acid with 10 mg/kg nicotinic acid alone or in combination with 100 mg/kg 2,5-DMP described above.

## BEST MODE FOR CARRYING OUT THE INVENTION

Hereinafter, the present invention will be explained in detail.

The present invention relates to a method of roasting coffee beans for providing coffee that is loved by many people as a favorite beverage and to a modified coffee obtained by the method. It is very preferable that, by contriving the method of roasting coffee beans, the component that is known to have an efficacy of drug by itself can be increased.

Further, the present invention relates to a supplement that contains components contained in coffee. It is very preferable that, by adjusting the intake or addition amount of the supplement, an efficacy of drug can be obtained for a prolonged time.

First, in the present invention, the method of roasting coffee beans to be used, for example, in the production of a modified coffee and a supplement is explained. However, the present invention is not limited thereto.

In the present invention, while the kind of the green coffee beans to be used is not particularly limited, however preferred are those green coffee beans that contain 300 mg or more of trigonelline in 100 g of the green beans, more preferably those green coffee beans that contain 400 mg or more of trigonelline in 100 g of the green beans, as described

above. Examples thereof include an Indonesian species, a Brazilian species, and a Columbian species, preferably one of Indonesian species and Brazilian species. A blend of a plurality of kinds of beans may also be used.

Referring to Fig. 3, roasting conditions in the present invention are explained.

As will be explained later in the section of EXAMPLE, Fig. 3 is a diagram showing results, expressed in ratio (%) with respect to the content of trigonelline, of determination of a nicotinic acid content obtained by roasting green coffee beans (Brazilian species) at 180 to 220˚C and determining every 10 minutes.

As will be apparent from Fig. 3, the maximum content of nicotinic acid is temperature-dependent. As a result of repeated experiments on the relationship between a temperature Y˚C and a time X (minute) in which the content of nicotinic acid reaches maximum at Y˚C, the following Formula 1 could be obtained.

Formula 1

$$X = 240 - Y$$

Wherein Y is 180 to 230˚C.

In the present invention, the roasting time of green coffee beans is a time in which a maximum amount (2 to 5 mg per 10 g of roasted beans) of nicotinic acid is produced at a temperature ranging from 180 to 230˚C mentioned above. From Formula 1, the roasting time that is preferable in the present invention is a function inversely proportional to the temperature. For example, the roasting time is 20 to 60 minutes in the range of 180 to 220˚C, 40 to 60 minutes in the range of 180 to 200˚C.

For example, since the constant of proportion is 1 in Formula 1, it is understood that when roasting is performed by starting at 180˚C with increasing the temperature at a constant rate until 220˚C at which the roasting is completed, roasting for 40 minutes can result in maximization of the amount of nicotinic acid produced.

Further, as will be apparent from Fig. 3, the amount of nicotinic acid produced at 180˚C or less is minute, which is not preferable for exhibiting the action of reducing blood lipid level. In addition, at this temperature, it takes a long time to increase the amount of nicotinic acid produced so that the Maillard reaction product had been produced evaporates during the roasting.

For example, Fig. 5-1 is a diagram showing a time course of amount of the Maillard reaction product generated at a roasting temperature of 200˚C as will be explained later in the section of EXAMPLE. As will be apparent from Fig. 5-1, the amount of the Maillard reaction product generated becomes maximum in 20 to 30 minutes and thereafter decreases.

Further, when the temperature exceeds 230˚C, carbonization of coffee beans proceeds, so that a longer time of roasting results in loss of taste.

Therefore, in the present invention, the roasting temperature is 180 to 230˚C for 15 to 60 minutes, preferably 200 to 230˚C for 15 to 25 minutes or 180 to 200˚C for 25 to 40 minutes, more preferably 200 to 220˚C for 15 to 25 minutes.

In the production of the supplement of the present invention, the roasting temperature is preferably 180 to 230˚C for 15 to 60 minutes, more preferably 200 to 230˚C for 15 to 25 minutes, still more preferably 200 to 220˚C for 15 to 25 minutes.

Under the above-mentioned roasting conditions, roasted coffee beans that contain 3 mg or more of nicotinic acid and 10 mg or more (preferably 30 mg or more) of the Maillard reaction product per 10 g of the roasted beans can be produced.

The roasting in the present invention is preferably dry roasting in order to apply heat to the coffee beans uniformly and constantly.

For the above-mentioned roasting, it is difficult to heat the whole coffee beans to the above-mentioned roasting temperature at a time uniformly. Accordingly, it is preferable that preheating at, for example, 100 to 150˚C be performed before the above-mentioned roasting. The time of preheating is preferably 5 to 20 minutes. In the case of a small amount of coffee beans (for example, about few hundreds grams), 5 minutes or less is sufficient.

Hereinafter, specific examples of compounds related to the effect of coffee on the prevention of, for example, development of diabetes are shown. In the present invention, the roasted coffee beans contain at least one group A compound (nicotinic acid compound) and at least one group B compound (Maillard reaction product) in higher concentrations than ordinary levels and are not limited to the examples described below.

In the production of the supplement of the present invention, the roasted coffee beans obtained as described above are preferable since they contain at least one group A compound (nicotinic acid compound) and at least one group B compound (Maillard reaction product) in higher concentrations than ordinary levels.

According to the present invention, the compounds that prevent the development and the like of type 2 diabetes are produced by the process of roasting coffee or derived from the produced compounds. Specifically, such compounds

include at least one group A compound (nicotinic acid compound) contained in the roasted coffee, at least one group B compound (Maillard reaction product), and at least one group C compound that is not contained in the roasted coffee but is produced in the body by metabolism after drinking coffee, for example, the modified coffee of the present invention.

Typical examples of the type B compounds that are known to be active components in the Maillard reaction product of the roasted coffee include fifteen kinds, more particularly, group B-1, group B-2, and group B-3 compounds. Those are respectively converted into group C-1, group C-2, and group C-3 compounds having a blood lipid level-reducing action by metabolic reaction. Preferred compounds among the group B compounds and the group C compounds are the group B-1 compounds and the group C-1 compounds, respectively.

Group A compounds (Nicotinic acid compounds)

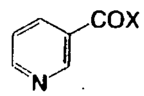

| Group A compounds | Kinds of X |
|---|---|
| 1a | -OH |
| 1b | $-NH_2$ |
| 1c | $-OCH_3$ |

Group B compounds (Maillard reaction products)

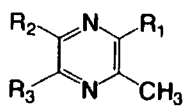

| Group B-1 compounds | $R_1$ | $R_2$ | $R_3$ |
|---|---|---|---|
| 2a | H | H | H |
| 2b | $CH_3$ | H | H |
| 2c | H | $CH_3$ | H |
| 2d | H | H | $CH_3$ |
| 2e | $CH_3$ | $CH_3$ | H |
| 2f | $CH_3$ | $CH_3$ | $CH_3$ |

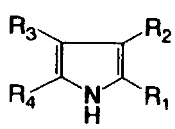

| Group B-2 compounds | $R_1$ | $R_2$ | $R_3$ | $R_4$ |
|---|---|---|---|---|
| 3a | CHO | H | H | H |
| 3b | $CH_3$ | H | H | H |
| 3c | $CH_3$ | $CH_3$ | H | H |
| 3d | $CH_3$ | H | $CH_3$ | H |
| 3e | $CH_3$ | H | H | $CH_3$ |

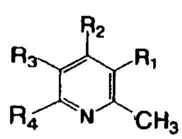

| Group B-3 compounds | $R_1$ | $R_2$ | $R_3$ | $R_4$ |
|---|---|---|---|---|
| 4a | H | H | H | H |
| 4b | $CH_3$ | H | H | H |
| 4c | H | $CH_3$ | H | H |
| 4d | H | H | $CH_3$ | H |
| 4e | H | H | H | $CH_3$ |

Group C compounds (Metabolites of Maillard reaction products)

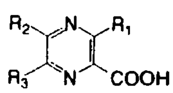

| Group C-1 compounds | $R_1$ | $R_2$ | $R_3$ |
|---|---|---|---|
| 5a | H | H | H |
| 5b | $CH_3$ | H | H |
| 5c | H | $CH_3$ | H |
| 5d | H | H | $CH_3$ |
| 5e | $CH_3$ | $CH_3$ | H |
| 5f | $CH_3$ | H | $CH_3$ |
| 5g | $CH_3$ | $CH_3$ | $CH_3$ |

(continued)

Group C compounds (Metabolites of Maillard reaction products)

| | Group C-2 compounds | $R_1$ | $R_2$ | $R_3$ | |
|---|---|---|---|---|---|
| 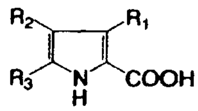 | 6a | H | H | H | |
| | 6b | $CH_3$ | H | H | |
| | 6c | H | $CH_3$ | H | |
| | 6d | H | H | $CH_3$ | |
| | Group C-3 compounds | $R_1$ | $R_2$ | $R_3$ | $R_4$ |
| 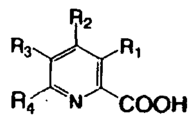 | 7a | H | H | H | H |
| | 7b | $CH_3$ | H | H | H |
| | 7c | H | $CH_3$ | H | H |
| | 7d | H | H | $CH_3$ | H |
| | 7e | H | H | H | $CH_3$ |

In the present invention, those compounds that are preferable from the viewpoint of the blood lipid level-reducing action among the compounds represented by the above formulae include nicotinic acid (1a), 2,5-dimethylpyrazine (2c), and pyrrole-2-aldehyde (3a), more preferably nicotinic acid (1a) and 2,5-dimethylpyrazine (2c).

The group A compounds, group B compounds (Maillard reaction products), and/or group C compounds (metabolites), contained in the supplement of the present invention, each are not particularly limited to those obtained from the above-mentioned roasted coffee beans but may be those produced by any one of organic synthesis techniques and biochemical techniques.

Since the modified coffee of the present invention contains at least one nicotinic acid compound and at least one Maillard reaction product, the slow-acting lipid level-reducing effect by the metabolite of the Maillard reaction product in liver complements the fast-acting lipid level-reducing effect by nicotinic acid.

The content molar ratio of the nicotinic acid/Maillard reaction product in the modified coffee of the present invention is preferably 1 nicotinic acid to 3 or more Maillard reaction products, more preferably 1 nicotinic acid to 5 or more Maillard reaction products, and still more preferably 1:10 to 1:20. Further, depending on the kind of the roasted coffee beans as a starting material and roasting conditions, the above-mentioned molar ratio can be set such that a minute amount (almost 0) of nicotinic acid to 100 mg or more Maillard reaction product/10 g of roasted coffee beans.

Here, it is for preventing the rebound phenomenon of nicotinic acid by the metabolite of the Maillard reaction product in liver that the amount of the Maillard reaction product is set 3 times larger or more than the amount of nicotinic acid as described later in the section of EXAMPLE. As a result, the low concentration of blood lipid can be maintained.

In the present invention, the method of grinding the roasted coffee beans is not particularly limited. The roughness of the ground beans is also not particularly limited. Moreover, there is no limitation on the method of extracting from the beans. Such ground bean powder may be used as it is as a blend component of blended coffee.

A coffee extract is one obtained by extracting ground powder of roasted coffee beans with water at any suitable temperature. In particular, to improve the recovery rate of the Maillard reaction product, ground bean powder may be extracted by a supercritical extracting method. From a similar point of view, any one of methods such as a column concentration method and a droplet alternating current partitioning method can be used.

The coffee extract can be provided either as it is without any processing as black coffee for beverage or as any types of coffee to which milk materials, sugars, flavors, and the like are added.

The coffee extract can be converted into powder by concentration under reduced pressure and freeze-drying, spray drying, or the like. An example of such powdered coffee includes instant coffee or the like filled in a bottle or a can.

Such powdered coffee having a coffee taste and preventive effects against diabetes and the like can be added as appropriate to any food and beverages such as coffee, milk, refreshing beverages, bread, and biscuit depending on the properties and purpose of each food or beverage during the production step or when being drunk or eaten.

From the viewpoints of reducing the concentration of the blood-free fatty-acid and reliably increasing the concentration of HDL-C, the total amount per day of the group A compound and the group B compound when drinking coffee is preferably 50 mg or more, more preferably 50 to 150 mg. However, the total amount per day of the group A compound and the group B compound may be a higher content of above 150 mg as far as the effect of the present invention is not detracted. Specifically, it is preferably that a cup of coffee contains 5 mg or more of nicotinic acid and 50 mg or more of the Maillard reaction product.

Hereinafter, a cup of coffee amounts to about 100 to about 150 ml and roasted bean powder necessary therefor

is 10 to 20 g although this amount may be greater or smaller than that amount.

The intake of the modified coffee of the present invention is preferably such that the sum of the group A compound and the group B compound is 50 to 150 mg per day. As compared with the critical maximum dose of nicotinic acid, i.e., 3 g per day (see Arch. Int. Med. 2004:164, 697-705), this amount corresponds to 60 times to 20 times less than the maximum critical dose of nicotinic acid and can be said to be a sufficiently safe content. Further, as compared with the critical minimum dosage of nicotinic acid (see Am. Heart J. 2002:143, 514-8), i.e., 50 mg/day, this amount can be said to be an amount sufficient to reduce the concentration of the blood-free fatty-acid and reliably increase blood HDL-C level.

It is long since known about nicotinic acids drugs that when the compound is taken in pharmacological amounts for a long time, an excessive amount administered per day increases the risk of developing side effects (such as face flush and a rebound phenomenon caused by nicotinic acids). It is preferable to describe necessary information directly on the container of the product in order to prevent the side effects since it does not only ensures safety of consumers but also reliably achieve the object of drinking the product.

The intake of the modified coffee of the present invention is preferably 5 mg or more per day as the nicotinic acid component (group A compound), more preferably 5 to 15 mg per day. However, the intake of the modified coffee of the present invention may be a higher content above 15 mg as far as the effect of the present invention is not detracted. Further, the sum of the nicotinic acid component and the group B compound is preferably 50 mg or more, more preferably 50 to 150 mg. However, the sum may be a higher content above 150 mg as far as the effect of the present invention is not detracted.

In the case of conventional coffees, it is shown that when a large amount of coffee (for example, 800 ml or more per day) is taken with a view to reducing the risk of diabetes, the risk of cardiovascular diseases increases (see J. Nutr. 134:2381-2386, 2004).

Since it is known that prevention of diabetes will result in a reduction in the risk of cardiovascular diseases, a causative agent for cardiovascular diseases due to drinking a large amount of conventional coffee is considered different from the component that prevents diabetes (for example, caffeine).

In the case of conventional coffees, the ratio of the amount of the Maillard reaction product to a standard amount of caffeine is 0.3 to 0.6 time by molar ratio whereas this molar ratio can be 2 times or more, preferably 3 to 5 times or more, in the case of the modified coffee of the present invention.

Therefore, when the modified coffee of the present invention is taken daily for a long time, the modified coffee of the present invention reduces the risk of cardiovascular diseases and prevents the development of diabetes.

Note that within the scope of the specification and claims, the term "standard amount of caffeine" means 90 to 150 mg in 10 g of roasted coffee beans.

The case where the above-mentioned group A to C compounds are added to any liquid foods and beverages (coffee, refreshing beverages, etc.) or hydrophilic solutions (for example, aqueous solutions and alcohol solutions) is described hereinbelow.

The nicotinic acid compounds of the group A are non-volatile and water-soluble and hence simple dissolution will suffice.

Since the group B compounds are water-soluble but have strong volatility, it is recommended that simple dissolution thereof may be performed after concentration of a coffee extract and freeze-drying, if any. As an exception of the group B compounds, the above-mentioned tetramethylpyrazine (2f) is hardly water-soluble, therefore it is recommended that tetramethylpyrazine be dissolved in a form of a salt such as phosphate.

Since the group C compounds are water-soluble but have weak volatility, they may be simply dissolved similarly to the group B compounds.

Next, the case where the above-mentioned group A to C compounds are added to any solid foods (bread, biscuit, etc.) or solid materials when producing or eating or drinking the supplement of the present invention is described hereinbelow.

In this case, it is preferable that solid (crystal) compounds of the group A and the group C be added. This is because the group B compounds are liquid or solids having low melting points and hence they are unstable as additives. When solid compounds are added, it is sufficient to simply add them and mix them well. When they are added to aqueous solution supplements, they may be simply dissolved in a final step of production.

Further, the supplement of the present invention may be any one of group A to C compounds singly or a mixture thereof. In this case, it is preferable that various improvements be made to a container and a storing method, such as prevention of moisture adsorption and use of desiccants.

From the viewpoint of complementing the fast-acting lipid level-reducing effect caused by nicotinic acid with the slow-acting lipid level-reducing effect caused by the metabolites of the Maillard reaction product in liver, it is preferable that the supplemented food of the present invention should contain a composition that contains at least one nicotinic acid compound and at least one Maillard reaction product. Therefore, it is preferable that the above-mentioned roasted coffee beans be used as a raw material for the supplemented food.

For example, the above-mentioned roasted coffee beans as the supplement of the present invention may be blended with any other coffee beans.

When the supplemented food of the present invention contains the above-mentioned composition, the nicotinic acid/Maillard reaction product content molar ratio of the food of the present invention is preferably 1 nicotinic acid to 3 or more Maillard reaction product, more preferably 1 nicotinic acid to 5 or more Maillard reaction product, and still more preferably 1:10 to 1:20. Further, depending on the kind of the roasted coffee beans as a starting material and roasting conditions, the above-mentioned molar ratio can be a minute amount (almost 0) of nicotinic acid to 100 mg or more Maillard reaction product/10 g of roasted coffee beans.

In the production of the supplement of the present invention, the method of grinding the roasted coffee beans is not particularly limited. The roughness of the ground beans is also not particularly limited. Moreover, there is no limitation on the method of extracting from the beans. Such ground bean powder may be used as it is as the supplement of the present invention or as a blend component of blended coffee. Alternatively, the food to which the ground bean powder is added as it is may be used as the supplemented food of the present invention.

Powder obtained by extracting ground powder of roasted coffee beans with water at any suitable temperature to obtain a coffee extract, concentrating the coffee extract under reduced pressure, and freeze-drying or spray drying the concentrate may be used as the supplement of the present invention. Also, food to which the powder is added may be used as the supplemented food of the present invention. The above-mentioned coffee extract may be used as the supplement of the present invention or food to which the above-mentioned coffee extract is added may be used as the supplemented food of the present invention.

In particular, to improve the recovery rate of the Maillard reaction product, ground bean powder may be extracted by a supercritical extracting method. From a similar point of view, any one of methods such as a column concentration method and a droplet alternating current distribution extraction method can be used.

The supplemented food of the present invention may be obtained by adding appropriately the above-mentioned powder or coffee extract to any food and beverages such as coffee, milk, refreshing beverages, bread, and biscuit depending on the properties and purpose of each food or beverage during the production step or when being drunk or eaten. On the other hand, the supplement of the present invention may be formulated into tablets, granules, powder, and the like by any formulating methods.

In particular, when the supplement of the present invention is added to coffee, various forms may be possible in compliance with the likes and tastes of the consumers. For example, the supplement of the present invention may be added in advance to a sweetener such as table sugar, a milk product such as cream, and the like that usually many people add to coffee. Alternatively, powder of the supplement of the present invention may be added singly. The coffee to which the supplement of the present invention is added is not particularly limited, and may be any one of black coffee, any type of coffee to which a milk materials, sugars, flavors and the like are added, powdered coffee obtained by concentrating and freeze-drying the coffee extract, and instant coffee filled in a bottle or a can. The supplement of the present invention is used by adding it to the coffees during production or when being eaten or drunk as appropriate. The appropriate addition when being eaten or drunk is also preferable from the viewpoint of adjusting the flavor generated when coffee beans are extracted with hot water with the supplement of the present invention. Furthermore, it is preferable to add the supplement of the present invention to refreshing beverages and tonic drinks as quasi-drugs other than coffee.

Next, an aspect in which the supplement of the present invention is added to coffee is explained. However, the present invention is not limited thereto.

From the viewpoints of reducing blood free-fatty acid level and reliably increasing HDL-C, it is preferable that when a cup of coffee is taken, the total amount of the group A compounds, the group B compounds, and/or the group C compounds be 50 to 100 mg. More particularly, it is preferable that a cup of coffee should contain 5 mg or more of nicotinic acid and 50 mg or more of the Maillard reaction product.

Here, it is for preventing the rebound phenomenon with the metabolite of the Maillard reaction product in liver as described later in the section of EXAMPLE that the amount of the Maillard reaction product is set at least 3 times the amount of nicotinic acid. As a result, the low blood lipid level can be maintained.

For example, the nicotinic acid compound and the Maillard reaction product contained in a cup of coffee (10 g of roasted coffee beans) are quantitatively assayed by any one of suitable methods, such as an NMR determination method and an HPLC method, and a deficit from 5 mg for nicotinic acid and a deficit from 50 mg for the Maillard reaction product can be supplied with the supplement of the present invention that contains nicotinic acid and the Maillard reaction product as a blend component. Further, for example, the supplement of the present invention may be formulated as follows. That is, only pyrazine carboxylic acid is selected from the group C compounds and a supplement that contains 25 mg of pyrazine carboxylic acid is formed and added.

Further, even when the content of nicotinic acid is 5 mg or less per a cup of coffee (10 g of roasted coffee beans), the supplement of the present invention may be added in a form of a supplement that contains only the metabolites of the Maillard reaction product (group C compounds) without containing neither the nicotinic acid compounds nor the Maillard reaction products.

It is preferable that the intake of the supplement of the present invention is such that the total amounts of the group A compounds, the group B compounds, and/or the group C compounds be 30 mg to 150 mg per day. As compared with the critical maximum dose of nicotinic acid, i.e., 3 g per day (see Arch. Int. Med. 2004:164, 697-705), this amount corresponds to 1/100 to 1/20 of the maximum critical dose and can be said to be a sufficiently safe content. Further, as compared with the critical minimum dosage of nicotinic acid (see Am. Heart J. 2002:143, 514-8), i.e., 50 mg/day, this amount corresponds to 1/1.7 to 3/1.7 and can be said to be an amount sufficient to reduce blood free-fatty acid level and reliably increase blood HDL-C level.

In the case of conventional coffees, the ratio of the amount of the Maillard reaction product to a standard amount of caffeine is 0.3 to 0.6 time by molar ratio whereas this molar ratio can be 2 times or more in the case of the modified coffee of the present invention. Preferably the molar ratio can be 3 to 5 times or even higher.

When the supplement of the present invention is added to such an amount of coffee that does not increase the risk of cardiovascular diseases (for example, 400 to 800 ml/day) as a blend component and the resultant is daily taken for a long time, the risk of cardiovascular diseases is reduced and the development of diabetes can be prevented.

Next, the mechanism in which the components in the modified coffee or supplement prevent the development of diabetes in the present invention is explained.

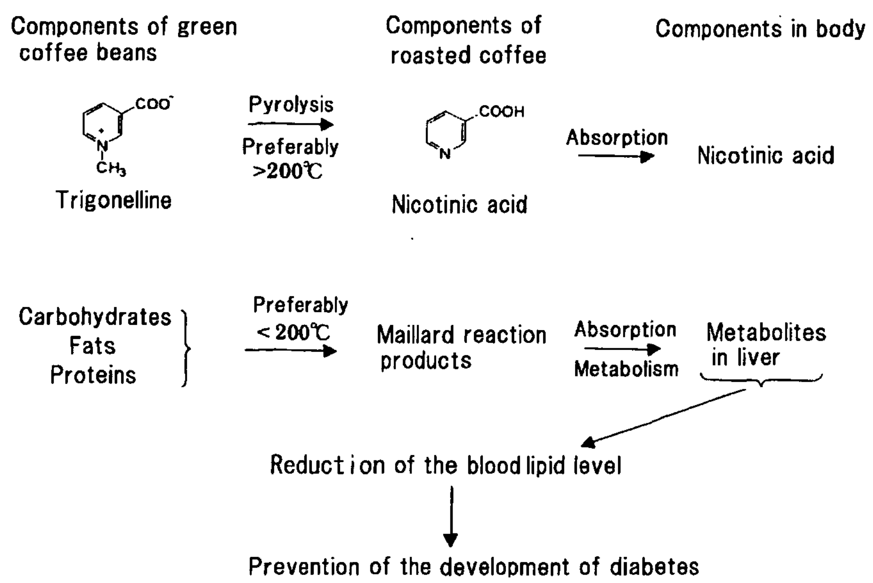


As described above, trigonelline contained in green coffee beans is converted into nicotinic acid by roasting and incorporated into the body as nicotinic acid. On the other hand, in the present invention, carbohydrates, fats, and proteins contained in green coffee beans are subjected to Maillard reaction upon roasting and the obtained reaction products are incorporated into the body, subjected to metabolism in liver, and their metabolites in liver reduce the blood lipid level, thus preventing the development of diabetes.

Fig. 1 shows the mechanism of blood lipid level-reducing action on molecular basis.

As shown in Fig. 1, the metabolites in liver of nicotinic acids (the above-mentioned group A compounds) and the Maillard reaction products (the above-mentioned group C compounds) are bound to HM74 receptors present on the membrane of adipose cells that constitute the adipose tissue (partly known). In this case, the group C compounds are ligands that more or less bind to HM74 receptors regardless of distinction among C1 to C3.

HM74 receptors to which the ligands are bound activate proteins and then inactivate adenyl cyclase (Ac). This suppresses biosynthesis of cAMP and PKA to inhibit the phosphorylation of hormonergic lipase. Finally, the hydrolysis rate of neutral fats reduces, resulting in a reduction in number of free-fatty acid and triglyceride released in blood from the adipose tissue. Though not shown in Fig. 1, the reduction in blood free-fatty acid level suppresses LDL synthesis in liver to promote HDL-C synthesis, thus improving systemic glucide/lipid metabolism in an individual who has an excess BMI (body mass index).

The relationship between the blood lipid level reduced and therapy/prevention of type 2 diabetes is described in detail in Yoshiya Hata, Ed., "Hypertriglyceridemia Handbook", pp148-160, Medicine Journal (1998); M. Lavezzari, et al., J. Int. Med. Res. 17:373-380 (1989); P. Tornvall, et al., J. Int. Med. 230:415-421 (1991), etc.

Next, a method of promoting health that involves a combination of the modified coffee or supplement of the present invention and a cholesterol-reducing drug therapy is explained.

The compounds that bind to the above-mentioned HM74 receptors, when taken in combination with statin-based drugs, increase blood HDL-C level as an effect that is not observed with the statin-based drugs alone (see, for example, Am. Heart J. 2002:143, 514-8; Am. J. Cardiol. 2004:93, 307-12; and Am. J. Cardiol. 2004:94, 306-11).

As described above with reference to Table 1, in the drug therapy of hyperlipidemia to those patients having lower HDL-C values than a standard value, it is expected that use of a combination of drugs I and II in Table 1 has the effect of reducing the blood total cholesterol value and increasing HDL-C, and based on this expectation clinical tests are performed.

Accordingly, similarly to the combination, the modified coffee or supplement of the present invention, not intended to perform drug therapy to patients, can be applied to, for example, healthy persons who are basically healthy or found to have a somewhat higher value of blood total cholesterol at the time of health checkup and hence take drug I alone. Table 2 shows a comparison between the drug therapy with a combination of the two drugs and the health-promoting method with the modified coffee or supplement of the present invention.

Table 2. Comparison between drug therapy with a combination of the two drugs and health-promoting method with the modified coffee or supplement of the present invention in hyperlipidemia

| Method | Combination of contents Reduction in total cholesterol level---Increase in HCL-C |
|---|---|
| Drug therapy with a combination of the two drugs | Statin based drug---Nicotinic acid (HM74 ligand) |
| Health-promoting method with the modified coffee or supplement of the present invention | Low-cholesterol food---Modified coffee or supplement of the present invention<br>Statin-based drug---Modified coffee or supplement of the present invention |

Such healthy persons can promote their health by ingesting the modified coffee or supplement of the present invention that contains a large amount of HM74 receptor ligands. In this case, when those who take drug I take the modified coffee or supplement of the present invention, the HDL-C-increasing effect can be exhibited potently. Further, even those healthy people who do not take drug I, if they perform health management such as limiting intake of food that contains a large amount of cholesterol, the intake of the modified coffee or supplement of the present invention results in that the HDL-C-increasing effect can be exhibited potently. In short, healthy people can achieve health-promoting effect that is of the same quality as that of the therapy with a combination of two drugs for patients suffering from hyperlipidemia by ingesting the modified coffee or supplement of the present invention.

When the present invention is used for this utility, the addition of the modified coffee or supplement of the present invention such that the daily intake as nicotinic acid is 50 mg or more provides a synergistic effect of increasing HDL-C besides the therapeutic effect by the drugs used in combination. Further, since the number of times of drinking coffee differs from person to person, to make the amount of the HM74-binding components per day constant, it is more effective to add the supplement of the present invention when drinking coffee than using the coffee that is produced with the addition of that component in advance.

Next, blended coffee roasted for different time periods will be explained as another aspect of the present invention. In addition, a supplement or a supplemented food having blended with coffee beans roasted under different conditions will be explained as another aspect of the supplement or the supplemented food of the present invention.

Conventional methods of roasting coffee mainly includes light roasting, medium roasting, and dark roasting. Components in coffee may vary or contents of the components may vary, even in the same component, depending on the roasting levels. Therefore, coffee beans roasted under different conditions contain different pharmacologically active components, respectively. Conventionally, the blended coffee is produced by blending green coffee beans having different characteristics such as variety, locality, and a shipping port in an appropriate proportion and roasting the blend of green coffee beans, and blending of coffee beans having roasted under different conditions has not been conducted. Therefore, the conventional blended coffee contains only a part of all of the pharmacologically active components of coffee, and contains few or no other components.

The blended coffee roasted for different time periods of the present invention and the supplement or the supplemented food having blended with coffee beans roasted under different conditions of the present invention each include a mixture of at least two kinds of roasted coffee beans selected from a plurality of roasted coffee beans in each of which an amount of any one of the following components (a) to (c) which promote health by preventing diabetes or the like is increased with respect to a certain amount of caffeine in a roasted coffee bean by changing a roasting condition:

(a) chlorogenic acid;
(b) chlorogenic lactone; and

(c) at least one nicotinic acid compound and at least one Maillard reaction product.

The blended coffee roasted for different time periods of the present invention and the supplement or the supplemented food having blended with coffee beans roasted under different conditions of the present invention each preferably include a mixture of a roasted coffee bean having the component (a) in an increased amount with respect to a certain amount of caffeine in the roasted coffee bean, a roasted coffee bean having the component (b) in an increased amount with respect to a certain amount of caffeine in the roasted coffee bean, and a roasted coffee bean having the component (c) in an increased amount with respect to a certain amount of caffeine in the roasted coffee bean, wherein the amounts of the components are increased by changing the roasting conditions.

There may be used a blend of at least two kinds of ground coffee powders or a blend of at least two kinds of coffee extracts, in each of which an amount of any one of the components (a) to (c) is increased with respect to a certain amount of caffeine in a ground bean. The variety, locality, or the like of the raw material beans may be the same, or may be different among the raw material beans.

The blended coffee roasted for different time periods of the present invention and the supplement or the supplemented food having blended with coffee beans roasted under different conditions of the present invention each can contain both of the components eliminated by the roasting and the components newly produced by the roasting in an optional proportion, and thus can concomitantly contain a plurality of components which cannot be yielded by the conventional blending method or by one roasting.

For example, 150 ml of a coffee extract can contain 30 mg or more of chlorogenic acid, 3 mg or more of a nicotinic acid compound, and 10 mg or more of a Maillard reaction product, and in addition thereto, 1 mg or more of chlorogenic lactone.

The blended coffee roasted for different time periods of the present invention and the supplement or the supplemented food having blended with coffee beans roasted under different conditions of the present invention each preferably include a mixture of at least two kinds of the following roasted coffee beans (d) to (f):

(d) a coffee bean obtained by roasting a green coffee bean as a raw material at 180 to 220˚C for 1 to 6 minutes (hereinafter, sometimes simply referred to as "light-roasted coffee bean"), or preferably at 180 to 210˚C for 1 to 5 minutes;

(e) a coffee bean obtained by roasting a green coffee bean as a raw material at 190 to 225˚C for 7 to 14 minutes (hereinafter, sometimes simply referred to as "medium-roasted coffee bean"), or preferably at 215 to 225˚C for 10 to 14 minutes; and

(f) a coffee bean obtained by roasting a green coffee bean as a raw material at 200 to 230˚C for 15 to 30 minutes (hereinafter, sometimes simply referred to as "dark-roasted coffee bean"), or preferably at 220 to 230˚C for 20 to 30 minutes.

The amounts of chlorogenic acid, chlorogenic lactone, and nicotinic acid and Maillard reaction products increase under the conditions of the light roasting (d), the medium roasting (e), and the dark roasting (f), respectively, with respect to the certain amounts of caffeine in roasted coffee beans.

For example, blending the roasted coffee beans (d) and (f) can increase the contents of chlorogenic acid, at least one nicotinic acid compound, and at least one Maillard reaction product with respect to the certain amount of caffeine in the roasted coffee beans. Further, blending the medium-roasted coffee beans (e) to the blend of the roasted coffee beans (d) and (f) additionally can increase the content of chlorogenic lactone with respect to the certain amount of caffeine in the roasted coffee beans.

A blend ratio is not particularly limited, but from a viewpoint of a content ratio of the respective components, is preferably represented by the following relationship: light-roasted:medium-roasted:dark-roasted = 0.5 to 1.5:2.5 to 3.5: 1.5 to 2.5.

Concomitant ingestion of a plurality of components which promote health by preventing diabetes or the like is expected to exert not only the effects of the respective components, but a synergistic effect as described below. For this purpose, it is preferable to use a product including as many kinds of active components having different pharmacological actions as possible.

The blended coffee roasted for different time periods of the present invention and the supplement and the supplemented food having blended with coffee beans roasted under different conditions of the present invention each concomitantly contain a plurality of components, and thus can exert the synergistic effect of and interaction between the components.

Now, pharmacologically known synergistic effects are described. When 2 drugs which exert the same efficacy of drug but have different actions are concomitantly used, the efficacy of drug is exerted at a level higher than a sum of the levels of the efficacies of drugs when the drugs are used independently. The synergistic effect is widely applied to clinical purposes. For example, acarbose which suppresses an excessively high blood glucose level after eating and a sulfonylurea drug which promotes secretion of insulin are often applied to slightly intractable diabetes.

Chlorogenic acid is known, by an animal test, to have a pharmacological action of reducing a speed of absorption

of glucose in digestive tracts (M. F. McCarty. A chlorogenic acid-induced increase in GLP-1 production may mediate the impact of heavy coffee consumption on diabetes risk. Med. Hypotheses 64: 848-853, 2005).

Enhancement of insulin sensitivity by chlorogenic lactone is described in J. Shearer, et al., Quinides of roasted coffee enhance insulin action in conscious rats. J. Nutr. 133: 3529-3532 (2003).

The inhibitory action of chlorogenic acid on the glucose absorption and the enhancing action of chlorogenic lactone on the insulin sensitivity are apparently and fully different from each other in terms of pharmacological action, and the efficacies of drugs thereof are complementary to each other. In other words, when the enhancement of the insulin sensitivity is caused concomitantly with suppression of the increase in blood glucose level after eating due to the inhibitory action, it is easy to suppress the slightly increased blood glucose level.

In addition, an interaction in which the rebound of nicotinic acid is resolved by Maillard reaction products is as described above.

Accordingly, the blended coffee roasted for different time periods of the present invention and the supplement or the supplemented food having blended with coffee beans roasted under different conditions of the present invention exert synergistically enhanced diabetes-preventing effects.

Hereinafter, the present invention will be explained in more detail with reference to examples. However, the present invention should not be considered to be limited thereto.

EXAMPLES

(1) HPLC analysis method of nicotinic acid contained in roasted coffee beans

Maillard reaction products, sources of aroma of coffee products, were produced at a roasting temperature of 200˚C. Then, the optimal roasting time for trigonelline in green coffee beans to be converted by heat into nicotinic acid was set to 40 minutes utilizing the Formula 1. Under the conditions, 200 g of green coffee beans (Brazilian species) was roasted and ground in a coffee mill for 20 seconds. 10 g of the obtained powder was immersed in 30 ml of hot water. The whole was centrifuged at 3,000 rpm for 5 minutes and 1 ml of the supernatant was poured into Sep-Pak PlusC18 (trade name, manufactured by Waters Corporation) and subjected to elution with 3 ml of acetonitrile/purified water (7/93). 5 μl of the eluate was analyzed by HPLC. The conditions of experiments were as follows. Using a liquid supply pump (trade name: BIP-1, manufactured by Jasco, Inc.), a multi-wavelength ultraviolet absorption detector (trade name: MULTI-320, manufactured by Jasco, Inc.), a data analysis microcomputer (trade name: Vectra386/20N, manufactured by Hewlett Packard), and an analytical column (trade name: Capcell PakC18ACR, manufactured by Shiseido, Ltd.), a mobile phase solvent (acetonitrile/pH 2.0 phosphate buffer (6/94)) was fed at a flow rate of 1 ml/minute.

Fig. 2 is a chromatogram obtained as a result of the analysis. In Fig. 2, "AU" in the vertical axis indicates unit of absorbance.

In Fig. 2, peak P1 indicates nicotinic acid. The UV absorption spectrum indicated by this peak completely corresponded to that of nicotinic acid standard preparation. When quantitative analysis was performed by a calibration curve method taking the dashed line as a base line, the content of nicotinic acid in 10 g of the roasted coffee beans used as a sample (mass of the roasted coffee beans corresponding to a cup of coffee) was 3.28 mg.

A few cups of the coffee with this content corresponded to the amount of nicotinic acid as vitamin required for one day. To achieve the daily dose as a therapeutic drug for hyperlipidemia (114 mg/day as the lower limit), 30 cups of coffee is necessary. However, a preventive effect of the modified coffee or supplement as a health-promoting food can be expected even with smaller amounts thereof.

Further, an epidemiologic research has revealed that drinking 7 cups of coffee per day led to a statistically significant decrease in the risk of the development of diabetes. The amount of nicotinic acid corresponding to 7 cups of coffee as shown in Fig. 2 was calculated to be 26 mg, which corresponded to 24% of the dose per day used in the therapy of hyperlipidemia. This suggests that a diabetes-preventing effect can be expected with the above-mentioned amount of coffee.

(2) Roasting time that maximizes production amount of nicotinic acid at any desired roasting temperature

By experimental operations similar to the HPLC method in the section (1) above, the roasting temperatures were set at intervals of 10˚C between 180 to 220˚C and the amounts of nicotinic acid were measured every 10 minutes, according to the roasting methods described in Agric. Biol. Chem. 49(12), 3467-3471, 1985 and Nutritional and Toxicological Consequence of Food Processing, 49-59, M. Friedman, Plenum Press, New York, 1991 Ed.

Fig. 3 is a graph showing the relationship between the content of nicotinic acid (mg) per 1 g of trigonelline contained in green coffee beans in the vertical axis and the roasting time in the horizontal axis.

As will be apparent from Fig. 3, the maximum content of nicotinic acid is temperature-dependent, and the above-mentioned Formula 1 could be led from Fig. 3 as an empirical formula showing the relationship between time X

(minute) in which nicotinic acid reaches the maximum proportion at temperature Y˚C.

(3-1) Roasting conditions suitable for producing Maillard reaction products

A sample obtained by being extracted from 20 g of coffee beans (Brazilian species) roasted at 200˚C for 20 minutes with 100 ml of hot water was measured in a 500-MHz NMR apparatus. Fig. 4-1 shows the obtained results. In Fig. 4-1, a group of signals of 2.2 to 2.5 ppm were assigned to the Maillard reaction products and contained, for example, a set of singlets assigned to the structure N=C-$CH_3$ common among the group B compounds. Fig. 4-2 shows a spectrum obtained by determination of the extract of an aqueous solution of coffee with heavy chloroform ($CDCl_3$). Fig. 4-3 is a partially enlarged diagram of the spectrum shown in Fig. 4-2 in the range of 2.0 to 2.5 ppm.

As will be apparent form Fig. 4-1, a signal assigned to pyrrole-2-aldehyde from among the group B compounds was confirmed. The content of pyrrole-2-aldehyde could be readily determined by NMR spectrum in contrast to other compounds of which the quantitative analysis was practically impossible. Accordingly, paying attention to N=C-$CH_3$, which is a partial structure common among the compounds, quantitative analysis by NMR spectrum was performed.

Standard preparations of the group B compounds were purchased and 0.5 mg of each of them was added to 1. 0 ml of aqueous solution of coffee and 500-MHz NMR was measured. All N=C-$CH_3$ signals were observed in the range of 2.2 to 2.5 ppm.

As will be apparent from Figs 4-2 and 4-3, approximate contents of the group B compounds could be estimated by comparing with the methyl group in caffeine that is a coexisting main component. Upon calculation using integrated values, the content of the group B compounds was 14 mg per 10 g of the roasted beans.

Further, a mixture of caffeine (non-odor) and the Maillard reaction products obtained by leaving the sample to stand after the above-mentioned $CDCl_3$-NMR determination so that $CDCl_3$ is volatilized gave an aroma unique to coffee (four subjects). On the other hand, the aqueous layer obtained after extraction of the above-mentioned aqueous solution of coffee with heavy chloroform ($CDCl_3$) had no such aroma of coffee.

Further, black coffee extracted from the coffee beans roasted at 200˚C for 20 minutes as described above was rich in a fruity aroma, had no bitter taste, and almost no sour taste. The aroma unique to coffee gradually increased when the coffee beans were roasted at 200˚C or more, or at 200˚C for 20 minutes or more. As a result, the coffee beans roasted at 220˚C for about 20 to about 25 minutes had good aroma and taste. On the other hand, when roasted at 230˚C for 25 minutes or more the roasted coffee beans showed scorched appearance and had increased bitter taste and sour taste and hence were not preferable.

(3-2) NMR quantitative analysis of nicotinic acid and caffeine in hot water having immersed roasted coffee beans

The case of roasting at 220˚C for 20 minutes that provided good aroma and good taste is explained.

10 g of Brazilian species coffee beans roasted at 220˚C for 20 minutes was cooled and then ground in a coffee mill for 20 seconds. 90 ml of hot water was added thereto and the whole was agitated well. A 10 ml portion of the mixture was taken in a centrifuge tube and centrifuged at 3,000 rpm for 5 minutes. Floating matters on the surface of the solution were removed and about 0.5 ml of the supernatant was transferred in an NMR tube, to which was added with 1 μl of aqueous 1% HCl solution and the resultant was determined for NMR spectrum at 500 MHz. Fig. 4-4 shows the spectrum representing the results. Fig. 4-5 is a partially enlarged diagram of the spectrum shown in Fig. 4-4 near 9 ppm. In Fig. 4-5, singlets of trigonelline (singlet pt) and nicotinic acid (singlet pn) assigned to 2-position protons were observed at 9.06 ppm and 8.95 ppm, respectively. 50 μl of nicotinic acid was added to this sample and a ratio of the integrated value of the obtained peak areas to the integrated value of Fig. 4-4 was calculated. Then, the amount of nicotinic acid per 10 g of the roasted coffee beans was calculated to be 3.28 mg. For example, to make the total amount of nicotinic acid 5 mg by an addition of nicotinic acid to the coffee beans, an addition of 1.72 mg of nicotinic acid per 10 g of the coffee beans is sufficient.

Further, in Fig. 4-4, calculation using the ratio of the integrated value of 2-position proton in nicotinic acid being 8.95 ppm and the integrated value of the methyl group proton in caffeine being 3.22 ppm afforded 147 mg as the amount of caffeine per 10 g of the roasted coffee beans.

(3-3) NMR quantitative analysis of Maillard reaction products in extract with hot water of roasted coffee beans

As described above, the set of singlets observed at 2.2 to 2.5 ppm shown in Fig. 4-4 are a group of signals of the partial structure common among the Maillard reaction products (for example, a methyl group adjacent to a nitrogen atom (N=C-$CH_3$)). The sample used in the determination shown in Fig. 4-4 was agitated in a vibrating mixer for 1 minute after addition of 0.5 ml of heavy chloroform, and then centrifuged at 3,000 rpm for 5 minutes to separate the heavy chloroform layer. The heavy chloroform layer was charged again in an NMR tube and determined for 500 MHz-NMR spectrum. Upon calculation of an integrated value of a group signals observed at 2.2 to 2.5 ppm in the obtained spectrum

in comparison with the integrated value of the signal of the methyl group of caffeine, the content of the Maillard reaction products in 10 g of the roasted coffee beans was 52 mg. As compared with the content of nicotinic acid in the section (3-2), this content corresponds to 15 times the content of nicotinic acid. Therefore, drinking this coffee can prevent the rebound phenomenon of nicotinic acid. In addition, when the content of nicotinic acid in this coffee is increased to 5 mg per 10 g of the coffee beans, the ratio is about 10 times as described in the section (3-2). Therefore, there is no need to add any more Maillard reaction product for the purpose of preventing the rebound phenomenon of nicotinic acid.

(3-4) Caffeine content/Maillard reaction product content molar ratio (comparison with conventional coffee)

Fig. 4-6 (a) is a partially enlarged diagram of the NMR spectrum at 2.2 to 3.5 ppm shown in Fig. 4-4. Fig. 4-6 (b) shows NMR spectrum (2.2 to 3.5 ppm) of a solution obtained by extracting with hot water a conventional commercially available roasted coffee made from Brazilian coffee beans as a raw material.

In Fig. 4-6 (a) and Fig. 4-6 (b), S1 and S1' designate a group of signals (for example, a set of singlets of methyl groups adjacent to nitrogen atoms in the Maillard reaction products) assigned to the Maillard reaction products observed at 2.2 to 2.4 ppm as described above. S2, S2', S3, and S3' designate signals of two methyl groups in caffeine.

A ratio of the signal intensity of S2 or S3 to the signal intensity of S1 (for the conventional commercially available roasted coffee, a ratio of the signal intensity of S2' or S3' to the signal intensity of S1') corresponds to the caffeine/ Maillard reaction product content molar ratio (molar ratio of methyl groups). In the case of the conventional commercially available roasted coffee in Fig. 4-6 (b), the ratio is about 1:0.8. Meanwhile, in the case of the roasted coffee of the present invention shown in Fig. 4-6 (a), the ratio is about 1:3.

Since the content of caffeine does not change by roasting, the modified coffee of the present invention shown in Fig. 4-6 (a) contains a relatively larger amount of the Maillard reaction products than the conventional commercially available roasted coffee, with the difference being 3.9 times. Drinking a coffee that has a relatively greater content of the Maillard reaction products than caffeine allows people to take a large amount of the Maillard reaction products while suppressing the intake of caffeine.

That is, the modified coffee or supplement of the present invention can decrease the influence of caffeine to the body while in contrast increasing the preferable action of nicotinic acid and the Maillard reaction products.

(3-5) Contents of the components of powdered coffee or supplement obtained by freeze-drying

10 g of the roasted coffee beans obtained in the section of (3-2) was ground and subjected to extraction with 90 ml of hot water. The obtained extract was freeze-dried and again dissolved in 90 ml of hot water. An about 0.5 ml portion of this was charged in an NMR tube and determined for NMR at 500 MHz. According to the sections (3-2) and (3-3), the contents of nicotinic acid and the Maillard reaction products were calculated to be 3.00 mg and 7.8 mg, respectively. Then, to increase the contents of nicotinic acid and the Maillard reaction products to, for example, 5 mg and 50 mg, respectively, it is only necessary to add 2 mg of nicotinic acid and 42.2 mg of the Maillard reaction products.

(3-6) Correlation between roasting time and the content of the Maillard reaction products

Subsequently, to know the influence of the roasting time at a roasting temperature of 200˚C, extraction with hot water by the same operation as described above and determination of NMR was performed every 5 minutes from the start of the roasting, and the ratio of the signal intensity assigned to the Maillard reaction products (in the region of 2.2 to 2.5 ppm) to the signal intensity of caffeine was obtained. Fig. 5-1 shows the relationship between the ratio and the roasting time.

As will be apparent from Fig. 5-1, almost no signals in the region of 2.2 to 2.5 ppm were observed in green beans. Roasting at 200˚C resulted in detection of a group of signals assigned to the Maillard reaction products. It was demonstrated that the yield of the Maillard reaction products at 200˚C reached maximum in 20 to 30 minutes. Referring to Fig. 3, maximum production time for nicotinic acid at 200˚C was 40 minutes. However, with respect to Fig. 5-1, in this time, the content of the Maillard reaction products rather decreases.

(3-7) Correlation between contents of respective components (caffeine, trigonelline, chlorogenic acid, chlorogenic lactone, nicotinic acid, and Maillard reaction products) and roasting time

5 coffee beans were taken out during roasting 3, 5, 10, 15, 20, 25, and 30 minutes after initiation of the roasting, respectively, and subjected to hot-water extraction by the same procedure as described above, and NMR spectra at 500 MHz were measured. For each of the spectra at respective time, a standard signal of caffeine was represented by a singlet of 3.28 ppm assigned to a methyl group, trigonelline was represented by a singlet of 9.06 ppm assigned to a proton at the second position thereof, chlorogenic acid was represented by a doublet of 6.75 ppm assigned to a proton

at the sixth position thereof, sucrose was represented by a doublet of 5.32 ppm assigned to a proton at the first position in glucose, chlorogenic lactone was represented by a singlet of 4.48 ppm derived from a proton of a methoxy group thereof, nicotinic acid was represented by a singlet of 8.95 ppm derived from a proton at the second position thereof, and Maillard reaction products were represented by a singlet of 2.37 ppm assigned to a $N{=}C{-}CH_3$ group thereof, and percentages of levels of the respective signals to the level of the caffeine signal were obtained. FIG. 5-2 shows the result.

As apparent from FIG. 5-2, the amounts of the components in green coffee beans decrease with time while new components were generated. Specifically, the decreased components include trigonelline, chlorogenic acid, and sucrose while the newly generated components include chlorogenic lactone, nicotinic acid, and Maillard reaction products. In a case of medium roasting of about 10 minutes, the amount of sucrose decreased to one tenth or less, indicating progression of Maillard reaction. The amounts of chlorogenic acid and trigonelline had already decreased to one half while chlorogenic lactone was generated by roasting for about 10 minutes, which corresponded to the medium roasting. Note that a sweet and mild aroma was obtained by roasting from 3 to 5 minutes after initiation of the roasting, which corresponded to the light roasting, and a rich aroma was obtained by roasting for about 10 minutes, which corresponded to the medium roasting.

Accordingly, by blending the light-roasted coffee, the medium-roasted coffee, and the dark-roasted coffee, for example, by blending coffee beans roasted for 5, 10, and 20 minutes, respectively, there can be provided modified coffee, and a coffee-like supplement or a coffee-like supplemented food, including chlorogenic acid, chlorogenic lactone, nicotinic acid, and Maillard reaction products in increased amounts with respect to a certain amount of caffeine in the roasted coffee beans.

(4) Metabolism of the Maillard reaction products in liver

Metabolic reaction of the Maillard reaction products in liver (1)

To confirm the metabolism of monomethylpyrazine represented by the following Formula 2, a kind of the Maillard reaction products, the following experiment was performed.

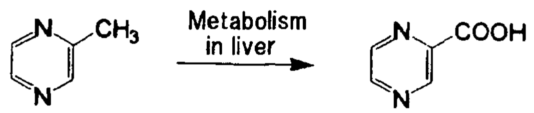


Monomethylpyrazine

Pyrazinecarboxylic acid

· · · · · Formula 2

Afour-week-old male Wistar rat weighing 150 g was orally administered with 7.5 mg (50 mg/kg) of monomethylpyrazine dissolved in physiological saline and thereafter urine of the rat was collected after 24 hours. A 5 μl portion of the urine was charged in an injection microsyringe (trade name: EXS-ODS, manufactured by Kusano Science) and injected with 100 μl of acetonitrile/phosphate buffer (7/93) adjusted to pH 2.0 into HPLC apparatus (specification and experimental conditions are the same as described above). Fig. 6 is a chromatogram of the obtained metabolites in the urine. In Fig. 6, peak P1 designates monomethylpyrazine administered, and peak P2 designates pyrazine carboxylic acid, which is the metabolite. Quantitative determination using a calibration curve method indicated a recovery rate in urine of 22.8%, of which about 90% was the metabolites.

That is, monomethylpyrazine, a Maillard reaction product, contained in coffee was converted into a group C compound having a blood lipid level-reducing effect by metabolism in liver.

Metabolic reaction of the Maillard reaction products in liver (2)

To confirm the metabolism of pyrrolealdehyde, represented by the following Formula 3, a kind of the Maillard reaction products, the following experiment was performed.

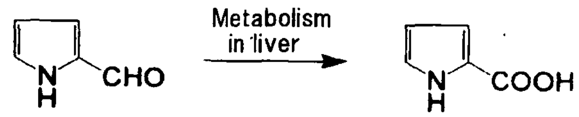


Pyrrole-2-aldehyde Pyrrole-2-carboxylic acid

· · · · · Formula 3

A four-week-old male Wistar rat weighing 150 g was orally administered with 7.5 mg (50 mg/kg) of pyrrole-2-aldehyde dissolved in physiological saline and thereafter urine of the rat was collected after 24 hours. A 5 μl portion of the urine was charged in an injection microsyringe (trade name: EXS-ODS, manufactured by Kusano Science) and the microsyringe was connected to an injector of HPLC apparatus. Then, 100 μl of acetonitrile/phosphate buffer (7/93) adjusted to pH 2.0 was injected into the HPLC apparatus through the injection microsyringe. The apparatus and the experimental conditions were the same as those described above. In Fig. 7, peak 2 is a chromatogram of the obtained metabolites in the urine. In Fig. 7, peak 1 designates to pyrrole-2-carboxylic acid, the metabolite of the administered compound. No peak corresponding to pyrrole-2-aldehyde (about 20 minutes) was detected. The recovery in urine was almost quantitative and the total amount of what was administered was metabolized.
That is, pyrrole-2-aldehyde, a Maillard reaction product contained in coffee, was converted into a group C compound having a blood lipid level-reducing action in a high efficiency by metabolism in liver.

Metabolic reaction of the Maillard reaction products in liver (3)

To confirm the metabolism of 2,5-dimethylpyrazine represented by the following Formula 4, a kind of the Maillard reaction products, the following experiment was performed.

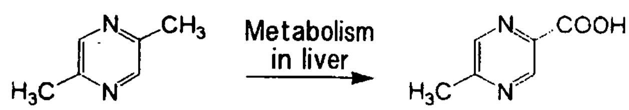


· · · · · Formula 4

2,5-dimethylpyrazine 5-methylpyrazine-2-carboxylic acid

Urine of a rat administered with 50 mg/kg of 2,5-dimethylpyrazine was collected after 24 hours and the amount of 5-methylpyrazine-2-carboxylic acid excreted was measured. The specification of the HPLC apparatus and experimental conditions were the same as described above.
Fig. 8 is a chromatogram of the obtained metabolites in the urine of the rat administered with 2,5-dimethylpyrazine. In Fig. 8, P1 designates 2,5-dimethylpyrazine administered and P2 designates 5-methylpyrazine-2-carboxylic acid, a metabolite thereof in liver.
As will be apparent from Fig. 8, besides peak P1 corresponding to the administered compound, peak 2 corresponding to the metabolite was observed in the urine. That is, 2,5-diemthylpyrazine, a Maillard reaction product contained in the urine, was converted into a group C compound having a blood lipid level-reducing action by metabolism in liver.

(5) Blood lipid level-reducing action by a metabolite of Maillard reaction product in liver

An experiment in which 7.5 mg (50 mg/kg) of 2,5-dimethylpyrazine, a kind of Maillard reaction product, was administered to a male Wistar rat weighing 150 g is exemplified. To observe time course of metabolism in liver of the compound represented by the above-mentioned Formula 4, blood was collected from a tail vein before the administration and every 1 hour after the administration, and blood levels of 2,5-dimethylpyrazine (2,5-DMP) and 5-methyl-pyrazine-2-carboxylic acid (5-MPCA) were determined.
That is, 10 μl of blood was collected at a time and centrifuged to separate plasma. A 5 μl portion of the plasma was injected into an injection microsyringe (trade name: EXS-ODS, manufactured by Kusano Science) and the microsyringe was connected to an injector of HPLC apparatus, through which 100 μl of acetonitrile/phosphate buffer (7/93) adjusted to pH 2.0 was injected into the HPLC apparatus. The specification of the apparatus and the experimental conditions were the same as those described above.
Fig. 9 shows a time course of blood levels of the compounds obtained by quantitative determination using a

calibration curve method. In Fig. 9, ♦ indicates 2,5-dimethylpyrazine and ■ indicates 5-methylpyrazine-2-carboxylic acid.

As will be apparent from Fig. 9, following the peak of the administered compound, the peak of the metabolite was observed in a delayed manner. That is, this delay affords slow-acting (as compared with nicotinic acid) blood lipid level-reducing effect to complement the fast-acting effect of nicotinic acid.

Further, using four-week-old male Wistar rats, the following experiment was performed. A control group was administered with physiological saline. A test group was administered with a solution of 50 mg/kg of 5-methylpyrazine-2-carboxylic acid (5-MPCA) dissolved in physiological saline. Immediately before starting the administration and 1, 2, 4, and 6 hours after the administration, each 6 rats were decapitated and blood was collected from each rat. According to a conventional method, plasma was separated from each sample blood and blood levels of free-fatty acid (FFA) were determined using a kit for determining free-fatty acid level. Fig. 10 shows the obtained results. That is, Fig. 10 is a diagram showing the effect of 5-methylpyrazinecarboxylic acid on the blood free-fatty acid level of the rats.

As will be apparent from Fig. 10, the most important feature is that no rebound phenomenon of blood free-fatty acid level was observed unlike nicotinic acid. When people drink coffee, 2, 5-dimethylpyrazine is subjected to metabolism in liver and converted into 5-MPCA, which then exhibits the effect.

(6) Blood lipid level-reducing action by a metabolite of Maillard reaction product in liver

In the development of drugs using nicotinic acid as a seed, it is long since known that pyrazine carboxylic acid N-oxides exhibit a blood lipid level-reducing action (see, for example, Eur. J. Med. Chem. 15: pp157-163 (1980)). Acipimox that exhibits the most potent action among them has been developed as a hyperlipidemia-treating drug (manufactured by Pharmacia in Italy, now Pfizer). During the development process, it has been considered that the action of a free base was weak.

In the present example, nicotinic acid, Acipimox, as well as pyrazine carboxylic acid and 5-methylpyrazine-2-arboxylic acid, metabolites in liver of the Maillard reaction products, were simultaneously experimented to compare their actions.

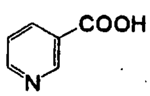
Nicotinic acid

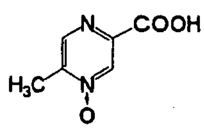
Acipimox

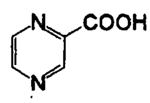
Pyrazinecarboxylic acid

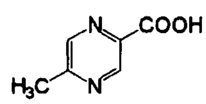
5-methylpyrazine-2-carboxylic acid

50 mg/kg of the above-mentioned four compounds were each administered to a group consisting of 5 male Wistar rats.

After 1 hour of the administration, each rat was decapitated and blood was collected. Then, blood free-fatty acid levels (Fig. 11 (a)) and blood triglyceride levels (Fig. 11 (b)) of sample bloods were determined by a conventional method. The results are shown in the graphs in Fig. 11 (a) and (b).

As will be apparent from Fig. 11 (a) and (b), as compared with the group administered with physiological saline, the groups administered with the compounds each exhibited a significantly different blood lipid level reduce while there was no significant difference among the compounds and the compounds exhibited almost equivalent effects. The blood lipid level reduced to 50% or less of that of the control group.

(7) Prevention of rebound phenomenon of nicotinic acid by Maillard reaction products (1)

By the same operation as that described in the section (6), each amount (control, 5, 10, 20, and 50 mg/kg) of nicotinic acid was orally administered to male Wistar rats weighing 150 g and blood was collected from the tail vein, followed by determination of time course of blood free-fatty acid level.

Fig. 12 is a graph showing a dose-dependent rebound phenomenon by nicotinic acid obtained by the above-mentioned operation.

As will be apparent from Fig. 12, in any dose of from 5 mg/kg to 50 mg/kg, the blood free-fatty acid level after 30 minutes of the administration was suppressed to 0.5 mEq/l or less. The time in which a rebound phenomenon was observed thereafter was different depending on the dose. The rebound phenomenon was observed after 1 hour with 5 mg/kg, after 2 hours with 10 mg/kg, after 2 to 3 hours with 20 mg/kg, and after 4 hours with 50 mg/kg. Thus, the developing time of nicotinic acid rebound phenomenon was dose-dependent and a positive correlation was established between the dose and time.

The results indicate that the rebound phenomenon of nicotinic acid observed in blood free-fatty acid level occurs owing to a short half-life of nicotinic acid in blood. That is, in the case of using nicotinic acid alone, when the blood nicotinic level that once increased becomes to reduce, the blood free-fatty acid level that was suppressed thus far abruptly increases in an inverted manner.

(8) Prevention of rebound phenomenon of nicotinic acid by Maillard reaction products (2)

By the same operation as that described in the section (6), 10 mg/kg of nicotinic acid alone or in combination with 100 mg/kg of 2,5-DMP as described above was orally administered to male Wistar rats weighing 150 g and time course of blood free-fatty acid levels were determined.

Fig. 13 is a graph showing a time course of blood free-fatty acid level when 10 mg/kg of nicotinic acid alone or in combination with 100 mg/kg of 2,5-DMP was used. In the graph, each point shows an average value of six experiments.

As will be apparent from Fig. 13, in the group administered with nicotinic acid alone, both individuals that showed a rebound phenomenon after 1 hour of the administration and individuals that showed no such phenomenon after 1 hour of the administration were present in a mixed manner, which indicated an interindividual difference. However, after 2 hours of the administration, all the animals showed the rebound phenomenon. In contrast, in the group administered with nicotinic acid in combination with 2,5-DMP, low levels of blood free-fatty acid were sustained after 30 minutes to 4 hours of the administration. That is, the use of 2,5-DMP in combination almost completely suppressed the rebound phenomenon and the pharmacological actions of nicotinic acid and 5-MPCA, a metabolite of 2,5-DMP, continued to exhibit in an additive manner.

Therefore, the Maillard reaction products in the modified coffee of the present invention are converted into corresponding metabolites in liver that have blood lipid level-reducing actions, and hence when people take the modified coffee of the present invention, a fast-acting blood lipid level-reducing effect due to nicotinic acid and a slow-acting blood lipid level-reducing effect due to the metabolites in liver of the Maillard reaction products are obtained without any rebound phenomenon.

Further, it is also apparent that when people take the supplement of the present invention, a fast-acting blood lipid level-reducing effect due to nicotinic acid and a slow-acting blood lipid level-reducing effect due to the metabolites in liver of the Maillard reaction products are obtained without any rebound phenomenon.

INDUSTRIAL APPLICABILITY

The modified coffee of the present invention is a coffee product having increased amounts of health-promoting components, and is preferably used as modified coffee for preventing diabetes and suppressing the risk of a cardiovascular disease by being drunk. In addition, the roasting method of the present invention is preferably used as a method of obtaining the modified coffee.

Further, the supplement and the supplemented food of the present invention are a supplement and a supplemented food containing roasted coffee components which are health-promoting components, and are preferably used as a supplement are a supplemented food for preventing diabetes and suppressing the risk of a cardiovascular disease by being drunk after being dissolved in coffee or by being eaten.

Having described our invention as related to the present embodiments, it is our intention that the invention not be limited by any of the details of the description, unless otherwise specified, but rather be construed broadly within its spirit and scope as set out in the accompanying claims.

This application claims priority on Patent Application No. 2004-289202 filed in Japan on September 30, 2004, Patent Application No. 2005-46258 filed in Japan on February 22, 2005, and Patent Application No. 2005-88882 filed in Japan on March 25, 2005, each of which is entirely herein incorporated by reference.

## Claims

1. A modified coffee containing 3 mg or more of a nicotinic acid compound and 10 mg or more of a Maillard reaction product per 10 g of roasted coffee beans.

2. The modified coffee according to the above claim 1, containing 30 mg or more of the Maillard reaction product.

3. The modified coffee, in which at least one of the nicotinic acid compound and at least one of the Maillard reaction product have respective contents in increased ratios as compared with a standard amount of caffeine from roasted coffee beans.

4. The modified coffee according to any one of claims 1 to 3, in which the nicotinic acid is represented by the following formula A,

Formula A

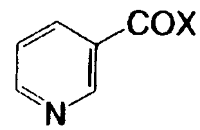

wherein X represents a hydroxyl group, an amino group or a methoxy group.

5. The modified coffee according to any one of claims 1 to 4, in which the Maillard reaction product contains at least one compound represented by the following formula B1, formula B2 or formula B3,

Formula B1

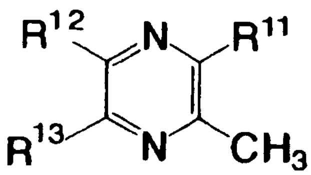

Formula B2

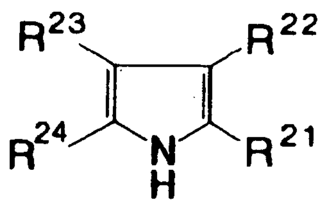

Formula B3

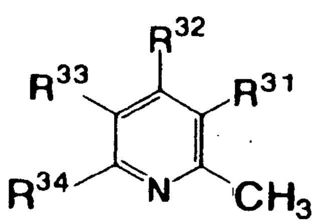

wherein, $R^{11}$ to $R^{13}$, $R^{21}$ to $R^{24}$, and $R^{31}$ to $R^{34}$ each represent a hydrogen atom or a methyl group, provided that $R^{21}$ may be an aldehyde group.

6. The modified coffee according to any one of claims 1 to 5, in which a coffee extract solution obtained from the roasted coffee beans is dried.

**7.** A method of roasting coffee beans, comprising roasting green coffee beans at 200 to 230˚C for 15 to 25 minutes.

**8.** A method of roasting coffee beans, comprising roasting green coffee beans at 180 to 200˚C for 25 to 40 minutes.

**9.** A method of roasting coffee beans, comprising roasting green coffee beans at a temperature of Y for a time of X satisfying the relationship represented by the following Formula 1:

Formula 1

$$X=240-Y$$

wherein Y is 180 to 220˚C and X is 20 to 60 minutes.

**10.** The method of roasting coffee beans according to claim 9, in which Y is 180 to 200˚C and X is 40 to 60 minutes.

**11.** The method of roasting according to any one of claims 7 to 10, in which the green coffee beans contain 300 mg or more of trigonelline in 100 g of the green beans.

**12.** The modified coffee obtained by the method of roasting according to any one of claims 7 to 11.

**13.** A modified coffee, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

**14.** A modified coffee, comprising at least two kinds of the following roasted coffee beans (d) to (f):

(d) a coffee bean obtained by roasting a green coffee bean as a raw material at 180 to 220˚C for 1 to 6 minutes;
(e) a coffee bean obtained by roasting a green coffee bean as a raw material at 190 to 225˚C for 7 to 14 minutes; and
(f) a coffee bean obtained by roasting a green coffee bean as a raw material at 200 to 230˚C for 15 to 30 minutes.

**15.** A modified coffee, comprising roasted coffee beans in each of which contents of chlorogenic acid, at least one nicotinic acid compound, and at least one Maillard reaction product are increased with respect to a certain amount of caffeine in the roasted coffee beans.

**16.** The modified coffee according to claim 15, wherein the mixture further comprises a roasted coffee bean including chlorogenic lactone in an increased amount with respect to a certain amount of caffeine in the roasted coffee bean.

**17.** A modified coffee, comprising 30 mg or more of chlorogenic acid, 3 mg or more of a nicotinic acid compound, and 10 mg or more of a Maillard reaction product per 150 ml of a coffee extract.

**18.** The modified coffee according to claim 17, further comprising 1 mg or more of chlorogenic lactone.

**19.** A coffee-like supplement containing at least one nicotinic acid compound, at least one Maillard reaction product, and/or at least one metabolite of the Maillard reaction product.

**20.** The supplement according to claim 19, in which the nicotinic acid compound is represented by the following formula A,

Formula A

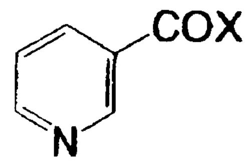

wherein X represents a hydroxyl group, an amino group or a methoxy group.

**21.** The supplement according to any one of claims 19 to 20, in which the Maillard reaction product contains at least one compound represented by the following formula B1, formula B2, or formula B3,

Formula B1

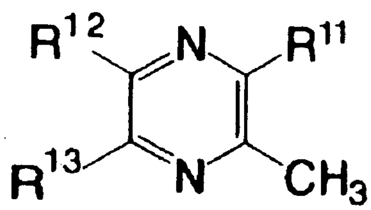

Formula B2

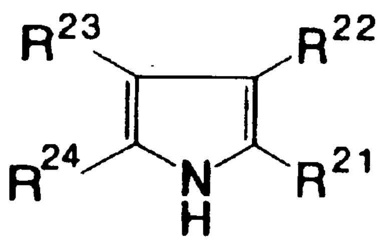

Formula B3

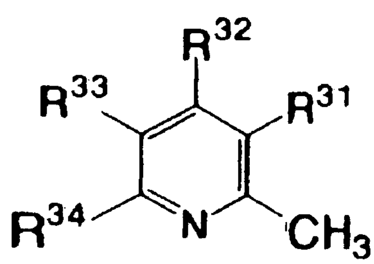

wherein $R^{11}$ to $R^{13}$, $R^{21}$ to $R^{24}$, $R^{31}$ to $R^{34}$ each represent a hydrogen atom or a methyl group, provided that $R^{21}$ may be an aldehyde group.

**22.** The supplement according to any one of claims 19 to 21, in which the Maillard reaction product contains at least one compound represented by the following formula C1, formula C2, or formula C3,

Formula C1

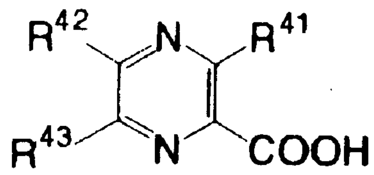

Formula C2

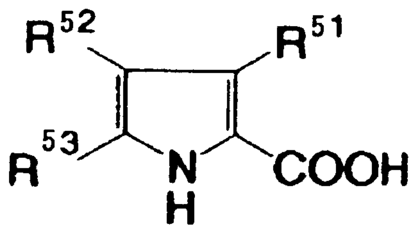

Formula C3

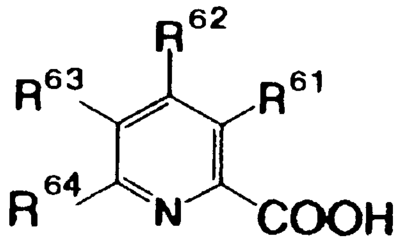

wherein $R^{41}$ to $R^{43}$, $R^{51}$ to $R^{53}$, $R^{61}$ to $R^{64}$ each represent a hydrogen atom or a methyl group.

**23.** The supplement according to any one of claims 19 to 22, in which the supplement is prepared from roasted coffee.

**24.** The supplement according to any one of claims 19 to 23, in which the nicotinic acid compound, the Maillard reaction product and/or the metabolite of the Maillard reaction product have respective contents in increased ratios as compared with a standard amount of caffeine obtained from the roasted coffee beans.

**25.** The supplement according to any one of claims 19 to 24, in which the supplement contains 3 mg or more of the nicotinic acid compound and a total 30 mg or more of the Maillard reaction product and the metabolite of the Maillard reaction product in a total 10 g of components obtained from the roasted coffee beans.

**26.** The supplement according to any one of claim 19 to 25, in which the supplement contains 3 mg or more of the nicotinic acid and at least 30 mg of the Maillard reaction product in a total 10 g of components obtained from the roasted coffee beans.

**27.** The supplement according to any one of clams 19 to 26, in which the nicotinic acid compound and the Maillard reaction compound are obtained from roasted coffee beans obtained by roasting green coffee beans at 200 to 230˚C for 15 to 25 minutes.

**28.** A coffee-like supplement, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

**29.** A coffee-like supplement, comprising at least two kinds of the following roasted coffee beans (d) to (f):

(d) a coffee bean obtained by roasting a green coffee bean as a raw material at 180 to 220˚C for 1 to 6 minutes;
(e) a coffee bean obtained by roasting a green coffee bean as a raw material at 190 to 225˚C for 7 to 14 minutes; and
(f) a coffee bean obtained by roasting a green coffee bean as a raw material at 200 to 230˚C for 15 to 30 minutes.

**30.** A coffee-like supplement, comprising a mixture of roasted coffee beans in each of which contents of chlorogenic acid, at least one nicotinic acid compound, and at least one Maillard reaction product are increased with respect to a certain amount of caffeine in the roasted coffee beans.

**31.** The coffee-like supplement according to claim 30, wherein the mixture further comprises a roasted coffee bean including chlorogenic lactone in an increased amount with respect to a certain amount of caffeine in the roasted coffee bean.

**32.** A coffee-like supplement, comprising 30 mg or more of chlorogenic acid, 3 mg or more of a nicotinic acid compound, and 10 mg or more of a Maillard reaction product per 10 g in total of components obtained from a roasted coffee bean.

**33.** The coffee-like supplement according to claim 32, further comprising 1 mg or more of chlorogenic lactone.

**34.** A coffee-like supplemented food containing at least one nicotinic acid compound, at least one Maillard reaction product and/or at least one metabolite product of the Maillard reaction product.

**35.** The supplemented food according to claim 34, in which the nicotinic acid is represented by the following formula A,

Formula A

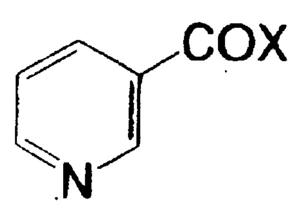

wherein X represents a hydroxyl group, an amino group, or a methoxy group.

**36.** The supplemented food according to claim 34 or 35, in which the Maillard reaction product contains at least one compound represented by the following formula B1, formula B2, or formula B3,

Formula B1

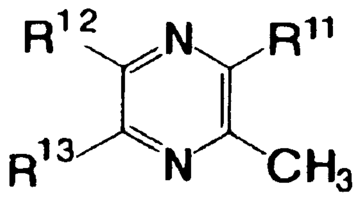

Formula B2

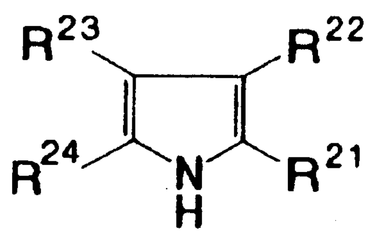

Formula B3

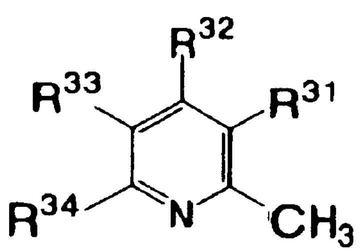

wherein $R^{11}$ to $R^{13}$, $R^{21}$ to $R^{24}$, $R^{31}$ to $R^{34}$ each represent a hydrogen atom or a methyl group, provided that $R^{21}$ may be an aldehyde group.

**37.** The supplemented food according to any one of claims 34 to 36, in which the Maillard reaction product contains at least one compound represented by the following formula C1, formula C2, or formula C3,

Formula C1

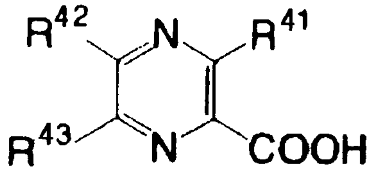

Formula C2

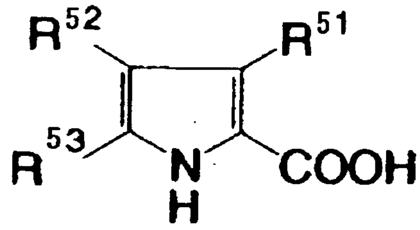

Formula C3

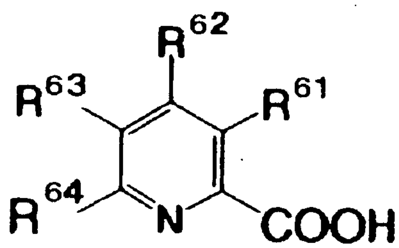

wherein $R^{41}$ to $R^{43}$, $R^{51}$ to $R^{53}$, $R^{61}$ to $R^{64}$ each represent a hydrogen atom or a methyl group.

**38.** The supplemented food according to any one of claims 34 to 37, in which the supplemented food is prepared from roasted coffee.

**39.** The supplemented food according to any one of claims 34 to 38, in which the nicotinic acid compound, the Maillard reaction product and/or the metabolite of the Maillard reaction product have respective contents in increased ratios as compared with a standard amount of caffeine derived from the roasted coffee beans.

**40.** The supplemented food according to any one of claims 34 to 39, in which the supplemented food contains 3 mg or more of the nicotinic acid compound and a total 30 mg or more of the Maillard reaction product and the metabolite of the Maillard reaction product in a total 10 g of components obtained from the roasted coffee beans.

**41.** The supplemented food according to any one of claims 34 to 40, in which the supplemented food contains 3 mg or more of the nicotinic acid and 30 mg or more of the Maillard reaction product in a total 10 g of components obtained from the roasted coffee beans.

**42.** The supplemented food according to any one of claims 34 to 41, in which the nicotinic acid compound and the Maillard reaction compound are obtained from roasted coffee beans obtained by roasting green coffee beans at 200 to 230°C for 15 to 25 minutes.

**43.** A coffee-like supplemented food, comprising at least two kinds of roasted coffee beans selected from a plurality of roasted coffee beans in each of which an amount of any one of the following components (a) to (c) is increased with respect to a certain amount of caffeine in a roasted coffee bean by changing a roasting condition:

(a) chlorogenic acid;
(b) chlorogenic lactone; and
(c) at least one nicotinic acid compound and at least one Maillard reaction product.

**44.** A coffee-like supplemented food, comprising at least two kinds of the following roasted coffee beans (d) to (f):

(d) a coffee bean obtained by roasting a green coffee bean as a raw material at 180 to 220°C for 1 to 6 minutes;
(e) a coffee bean obtained by roasting a green coffee bean as a raw material at 190 to 225°C for 7 to 14 minutes; and
(f) a coffee bean obtained by roasting a green coffee bean as a raw material at 200 to 230°C for 15 to 30 minutes.

**45.** A coffee-like supplemented food, comprising roasted coffee beans in each of which contents of chlorogenic acid, at least one nicotinic acid compound, and at least one Maillard reaction product are increased with respect to a certain amount of caffeine in the roasted coffee beans.

**46.** The coffee-like supplemented food according to claim 45, wherein the mixture further comprises a roasted coffee bean including chlorogenic lactone in an increased amount with respect to a certain amount of caffeine in the roasted coffee bean.

**47.** A coffee-like supplemented food, comprising 30 mg or more of chlorogenic acid, 3 mg or more of a nicotinic acid compound, and 10 mg or more of a Maillard reaction product per 10 g in total of components obtained from a roasted coffee bean.

**48.** The coffee-like supplemented food according to claim 47, further comprising 1 mg or more of chlorogenic lactone.

*Fig. 1*

NICOTINIC ACID

METABOLITE OF MAILLARD REACTION PRODUCTS IN LIVER

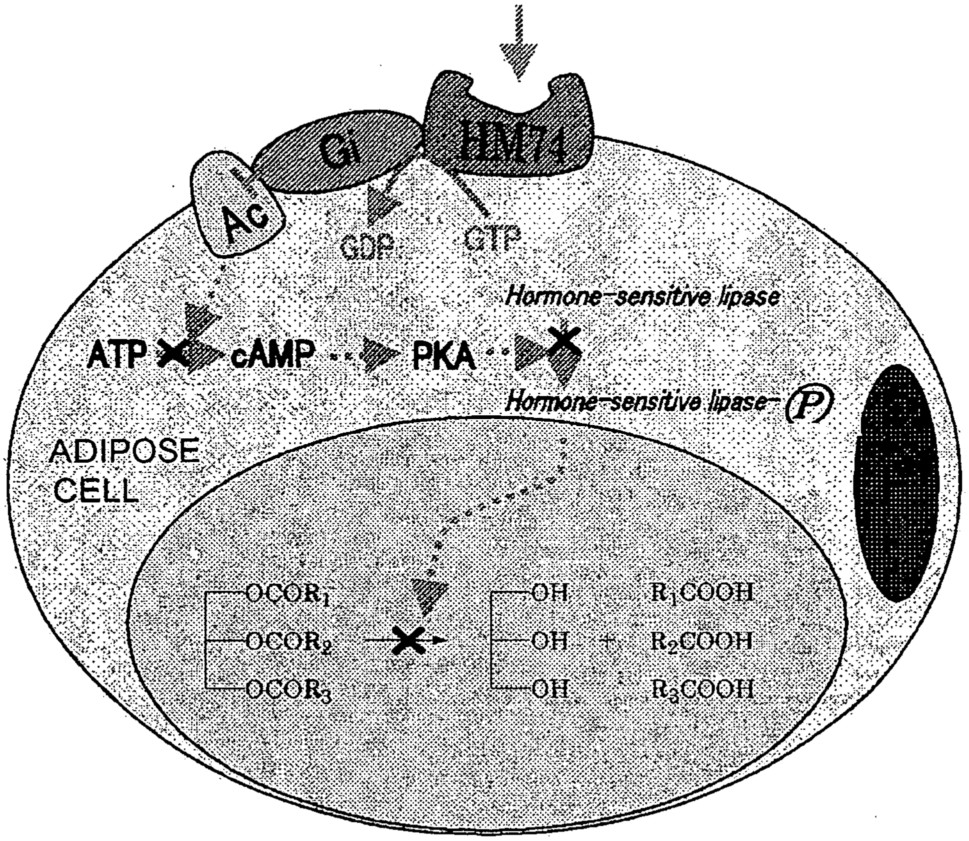

Fig. 2

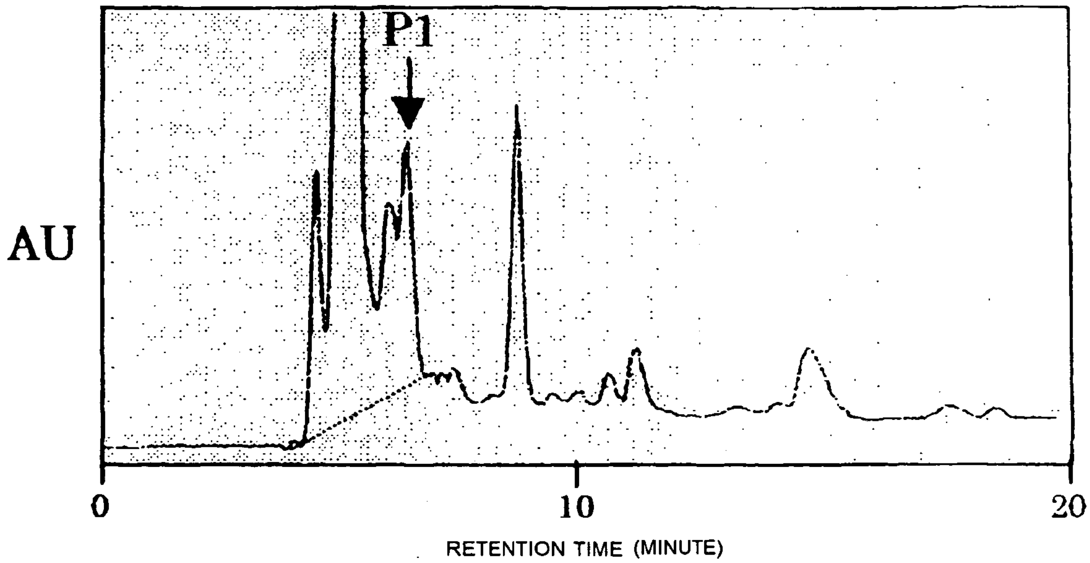
P1
AU
0
10
20
RETENTION TIME (MINUTE)

Fig. 3

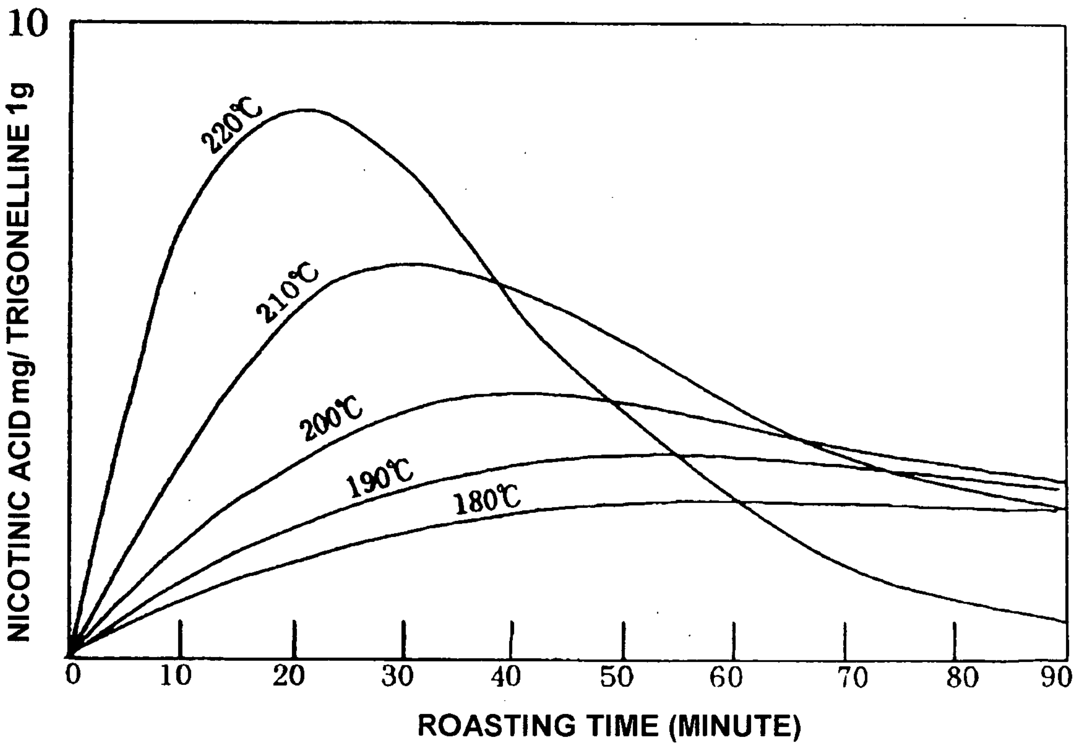
10
NICOTINIC ACID mg/ TRIGONELLINE 1g
220℃
210℃
200℃
190℃
180℃
0
10
20
30
40
50
60
70
80
90
ROASTING TIME (MINUTE)

*Fig. 4—1*

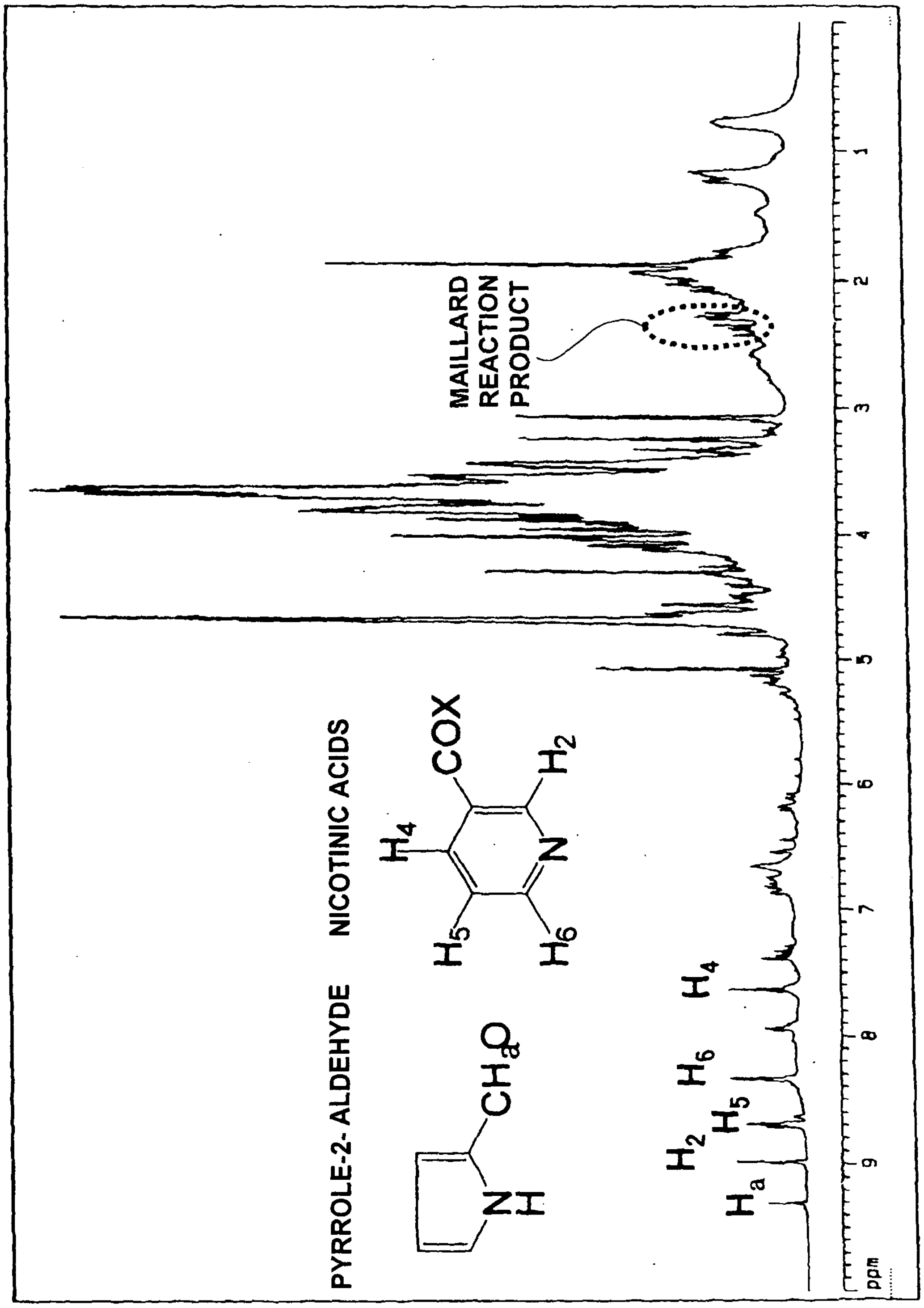
PYRROLE-2- ALDEHYDE
NICOTINIC ACIDS
MAILLARD REACTION PRODUCT
Ha H2 H5 H6 H4
ppm
9
8
7
6
5
4
3
2
1

Fig. 4-2

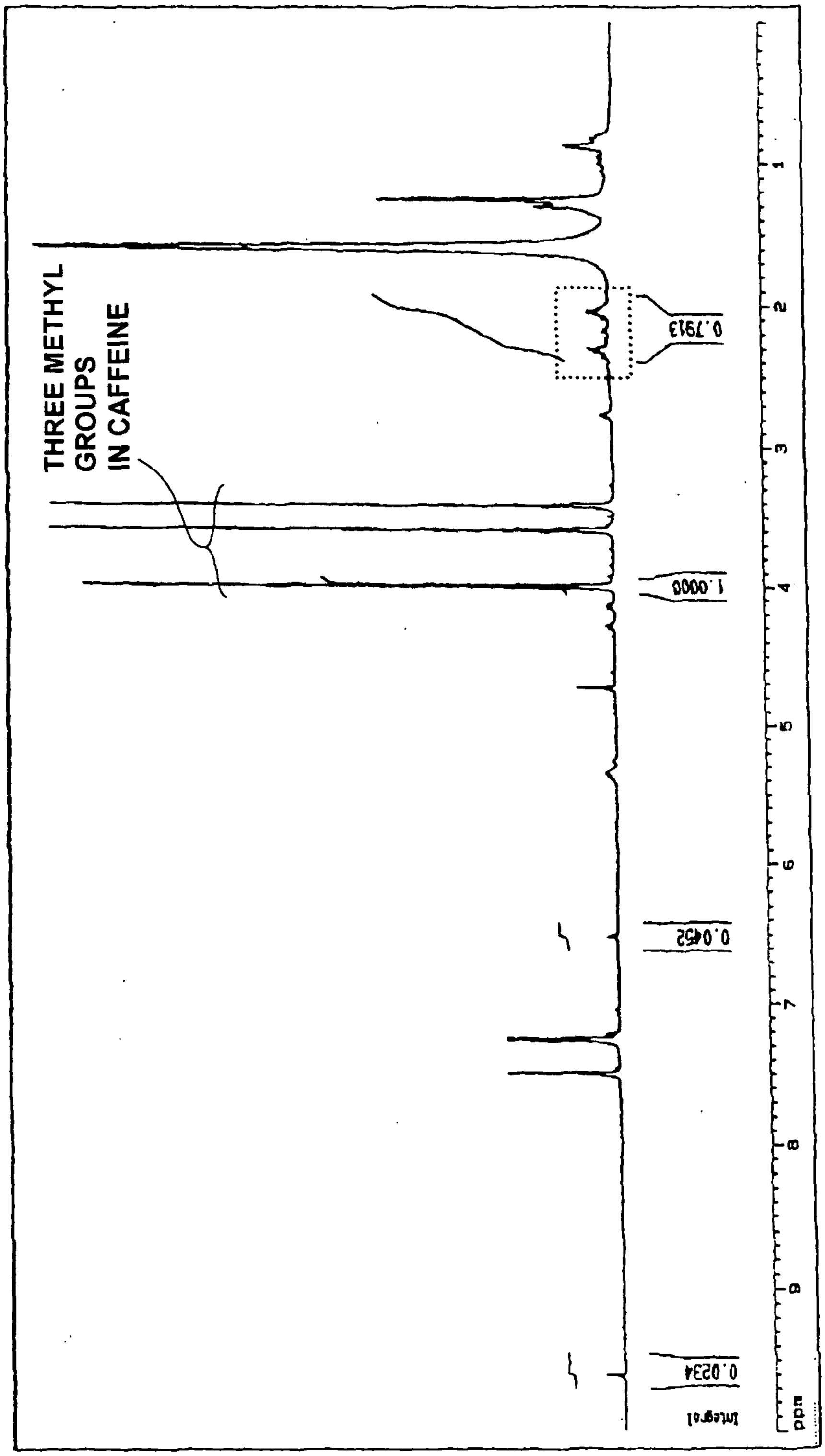
THREE METHYL
GROUPS
IN CAFFEINE
0.7913
1.0000
0.0452
0.0234
Integral
ppm

Fig. 4—3

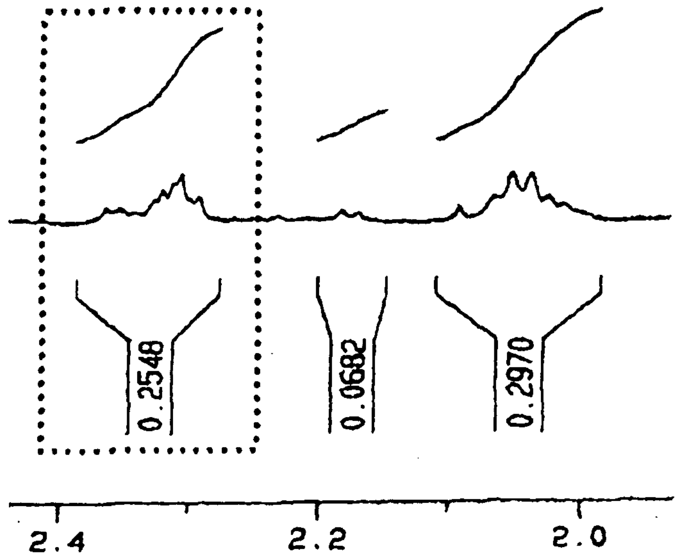
0.2548
0.0682
0.2970
2.4
2.2
2.0

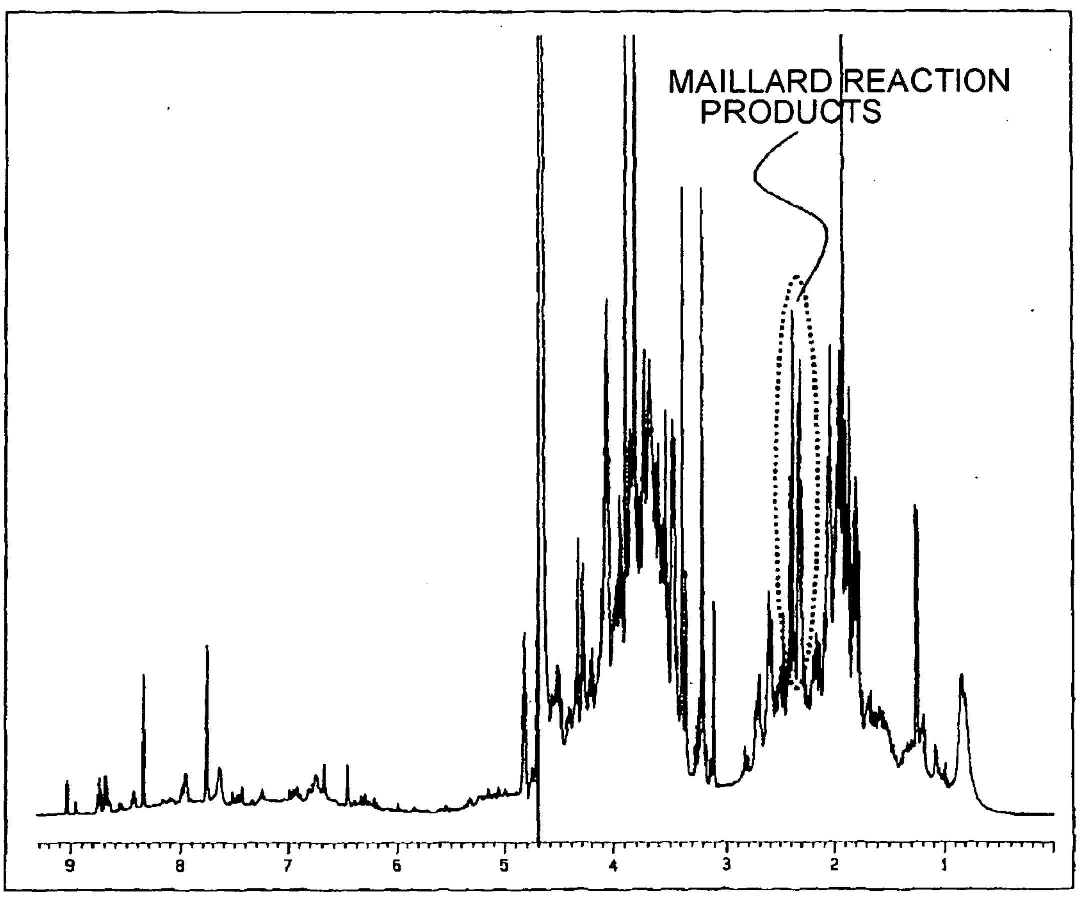
MAILLARD REACTION PRODUCTS
9
8
7
6
5
4
3
2
1

Fig. 4—4

Fig. 4-5

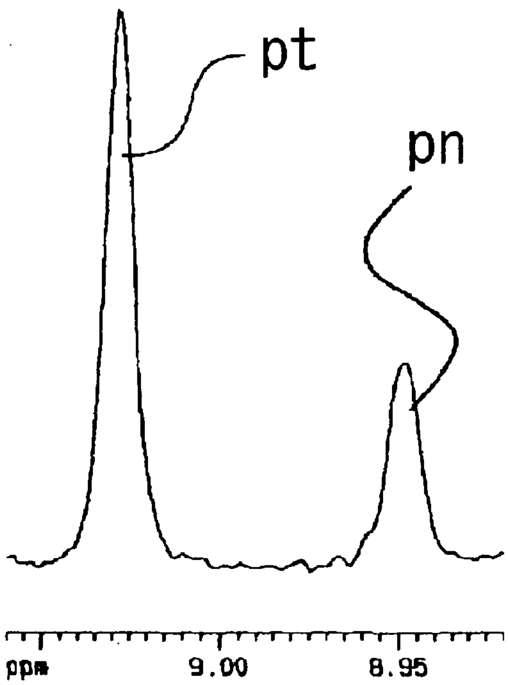


Fig. 4-6 (a)

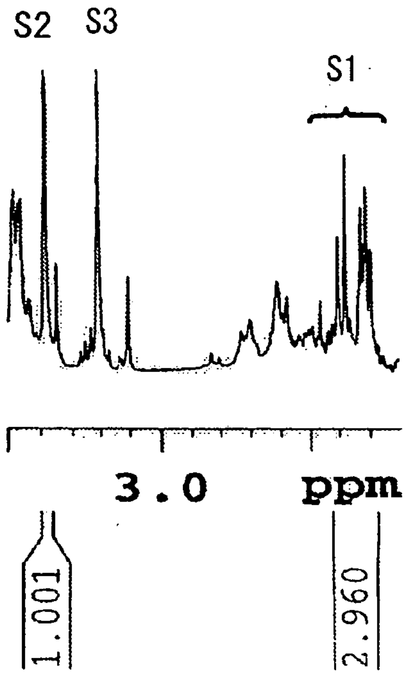


Fig. 4-6 (b)

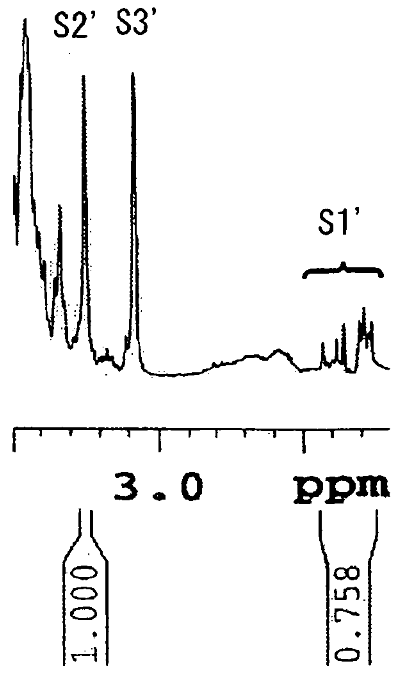

Fig. 5-1

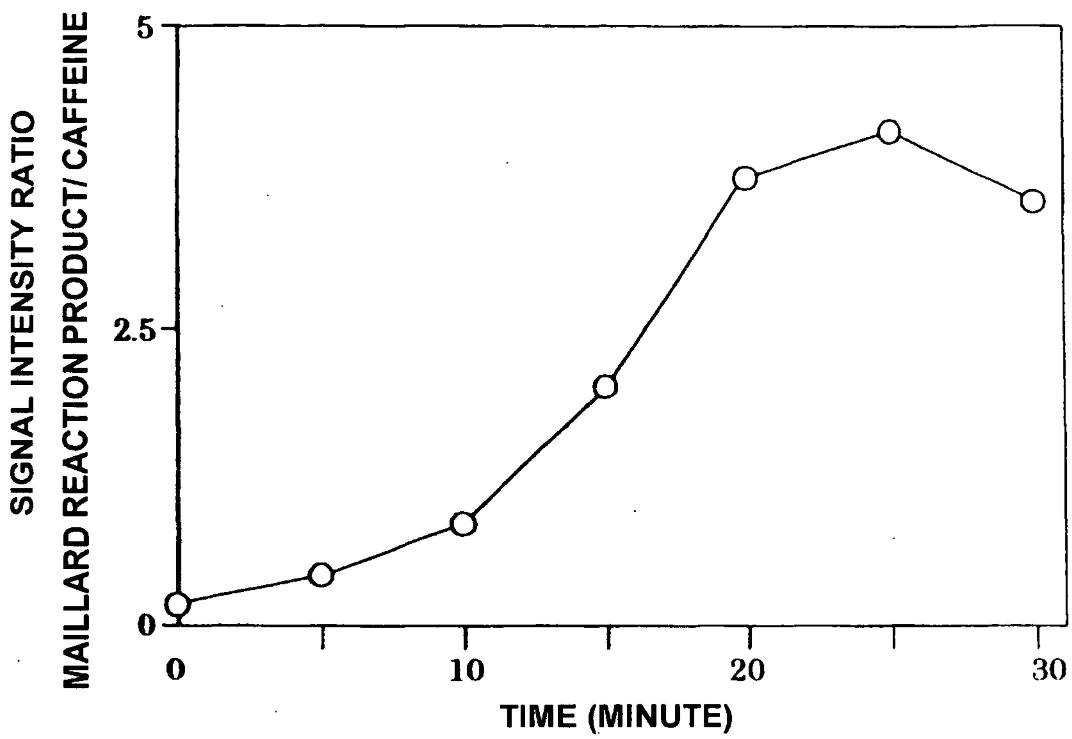
SIGNAL INTENSITY RATIO
MAILLARD REACTION PRODUCT/ CAFFEINE
5
2.5
0
0
10
20
30
TIME (MINUTE)

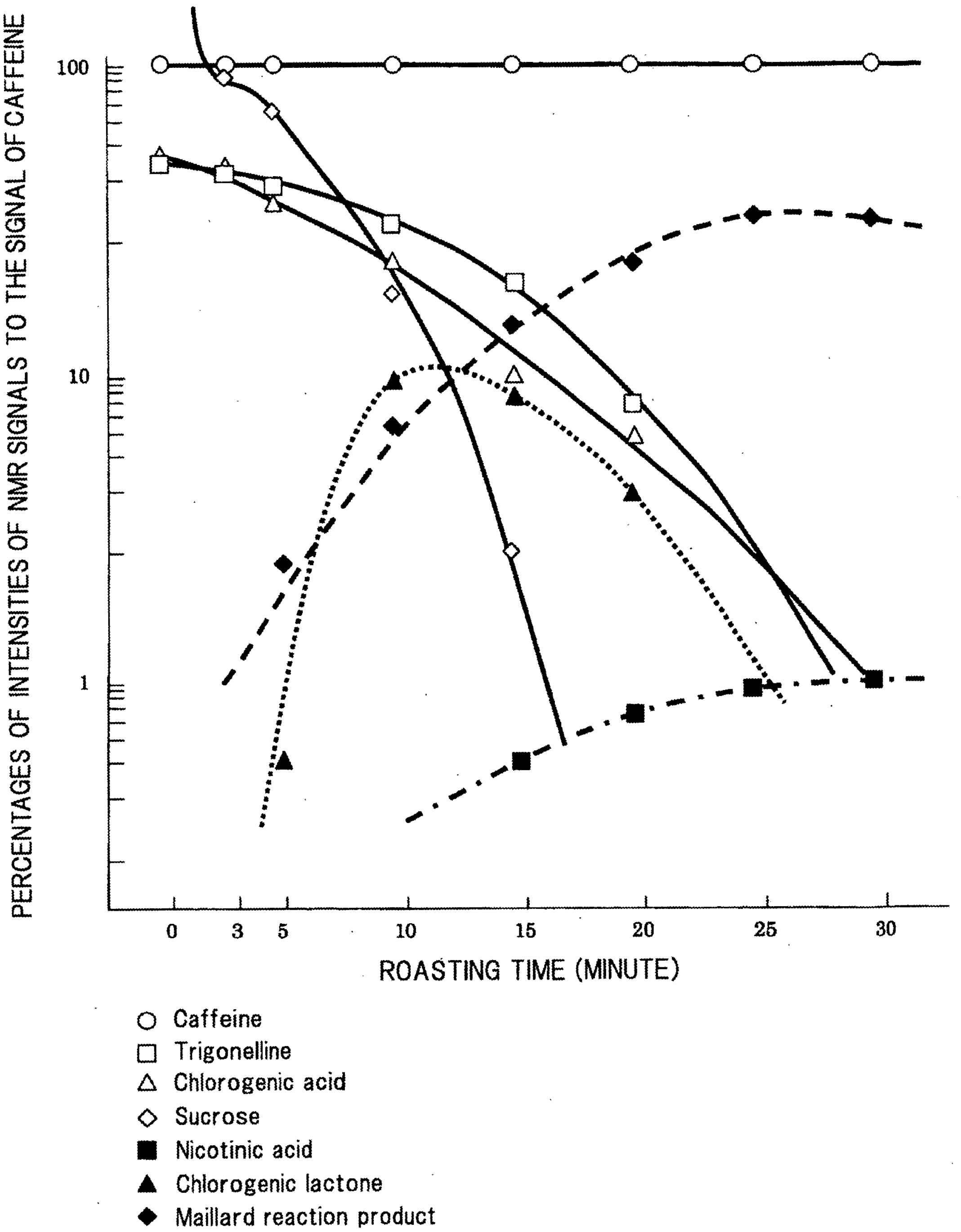
PERCENTAGES OF INTENSITIES OF NMR SIGNALS TO THE SIGNAL OF CAFFEINE
100
10
1
0
3
5
10
15
20
25
30
ROASTING TIME (MINUTE)
Caffeine
Trigonelline
Chlorogenic acid
Sucrose
Nicotinic acid
Chlorogenic lactone
Maillard reaction product

Fig. 5-2

Fig. 6

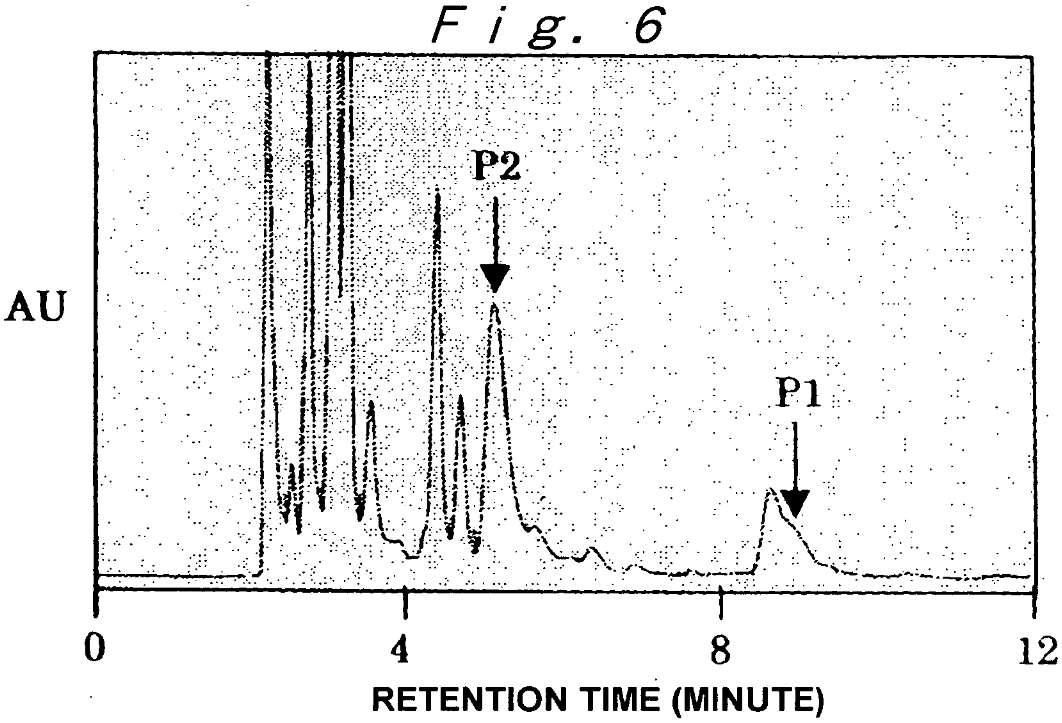
P2
AU
P1
0
4
8
12
RETENTION TIME (MINUTE)

Fig. 7

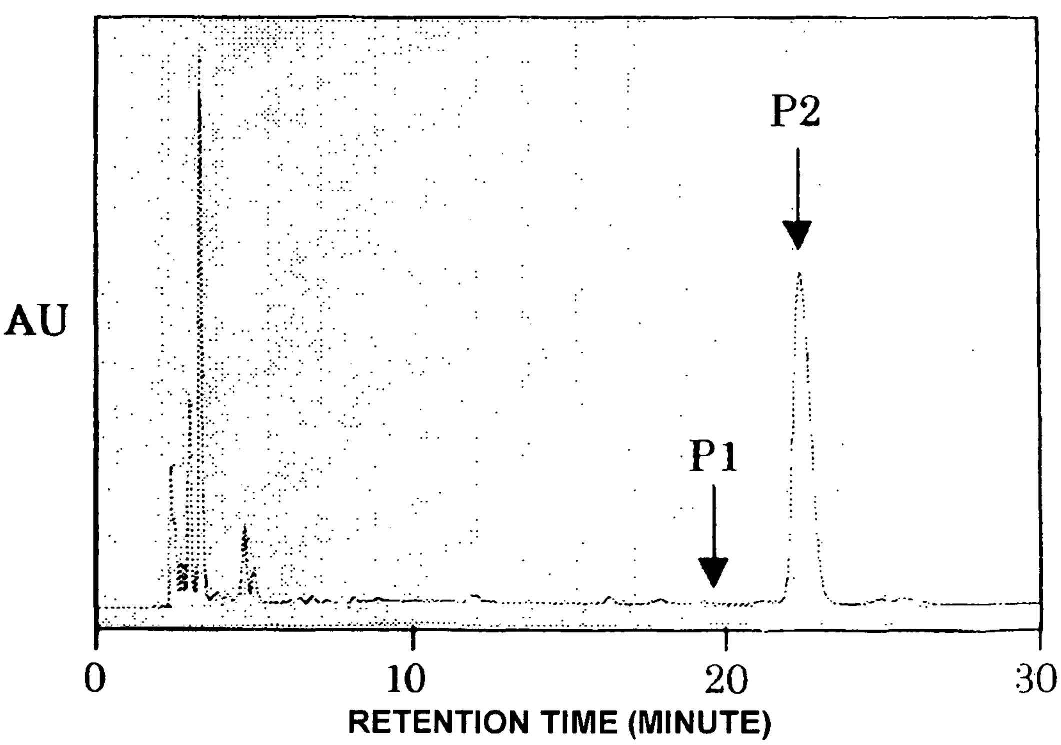
P2
AU
P1
0
10
20
30
RETENTION TIME (MINUTE)

*Fig. 8*

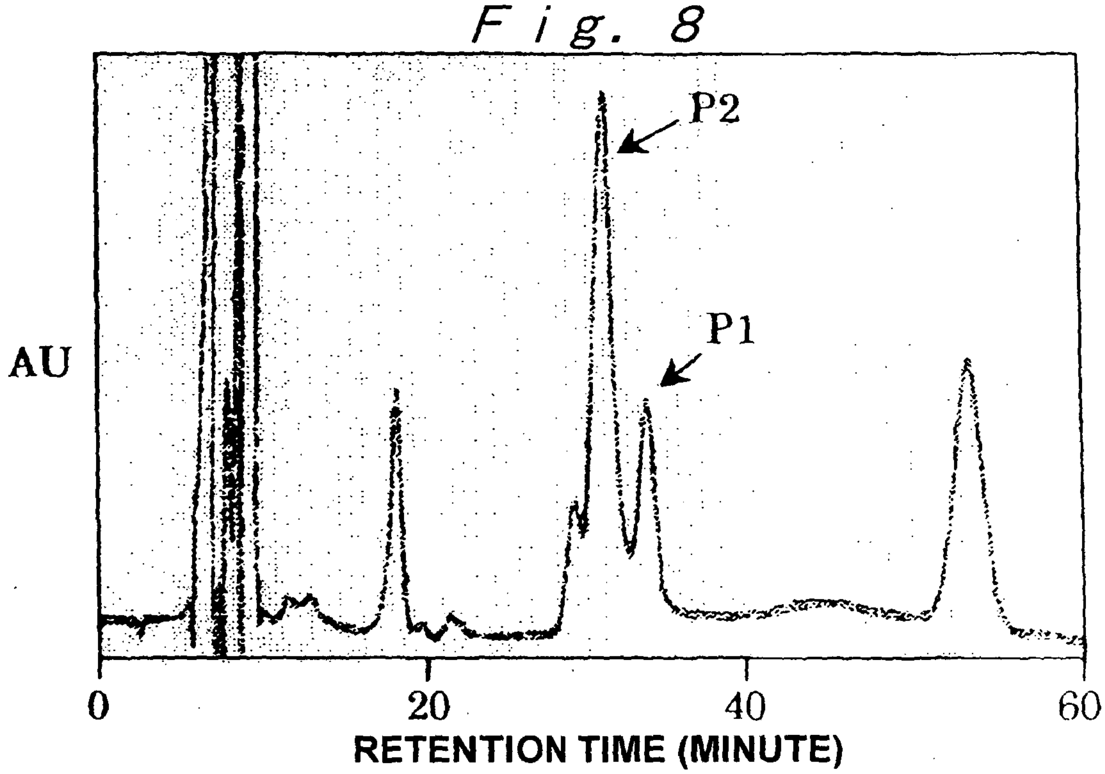
P2
P1
AU
0
20
40
60
RETENTION TIME (MINUTE)

*Fig. 9*

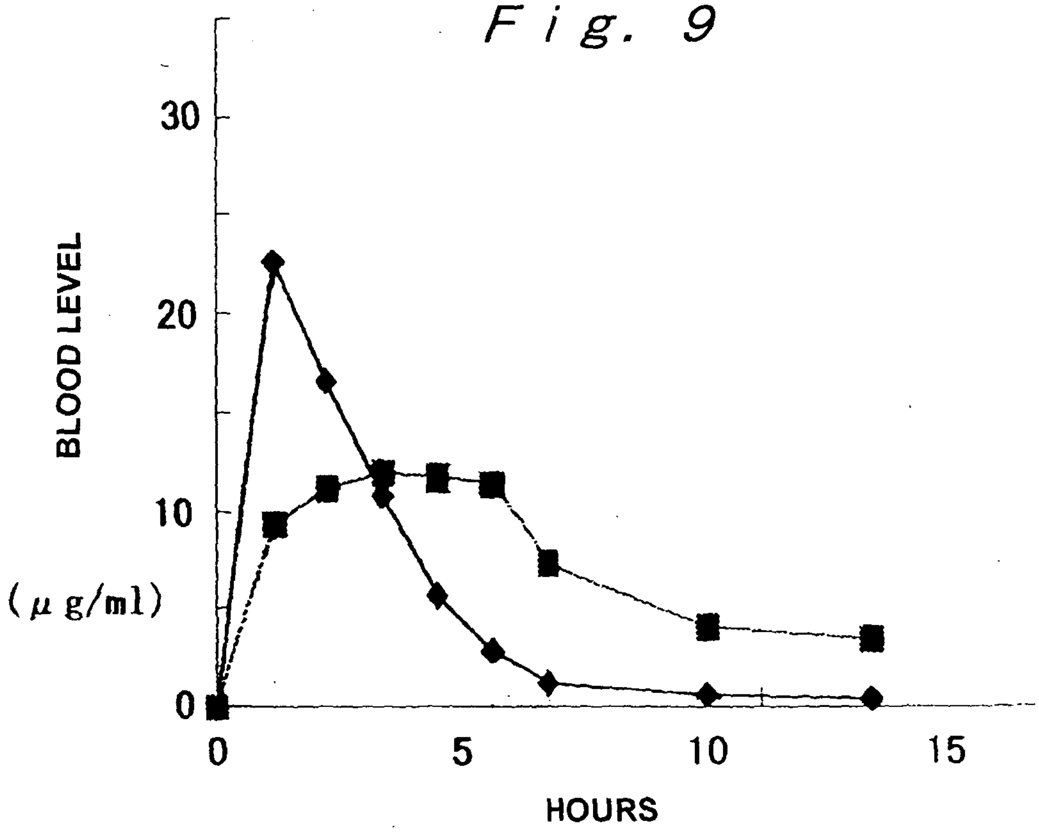
30
20
10
0
BLOOD LEVEL
(μ g/ml)
0
5
10
15
HOURS

Fig. 10

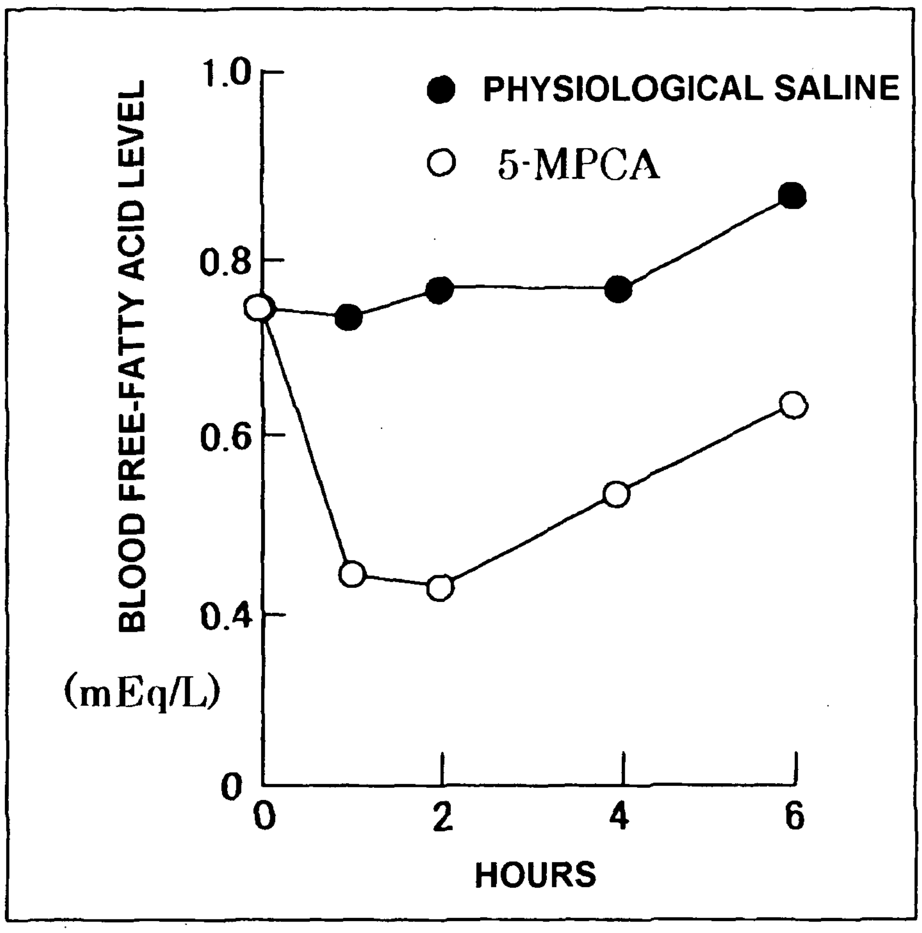
PHYSIOLOGICAL SALINE
5-MPCA
BLOOD FREE-FATTY ACID LEVEL
(mEq/L)
1.0
0.8
0.6
0.4
0
0
2
4
6
HOURS

Fig. 11 (b)

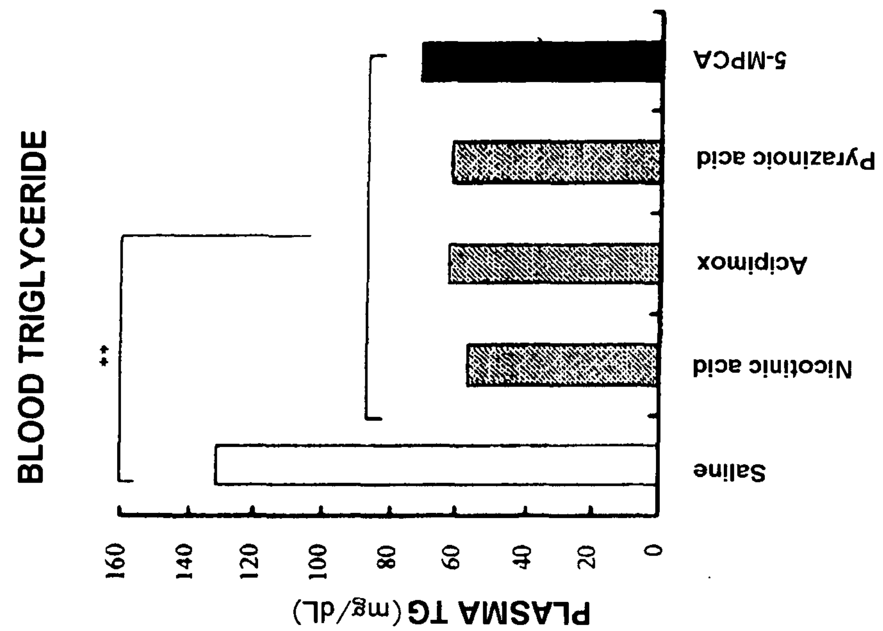


Fig. 11 (a)

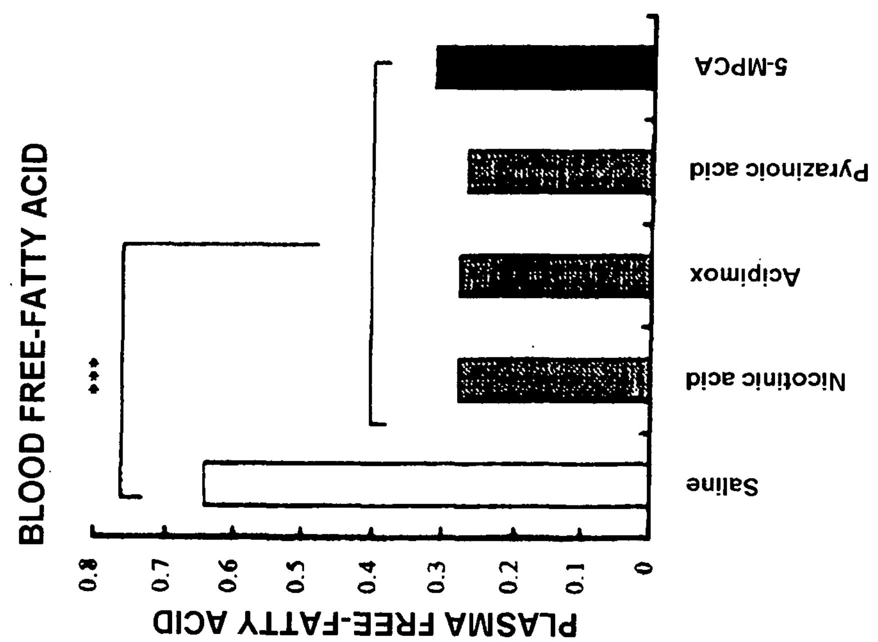

Fig. 12

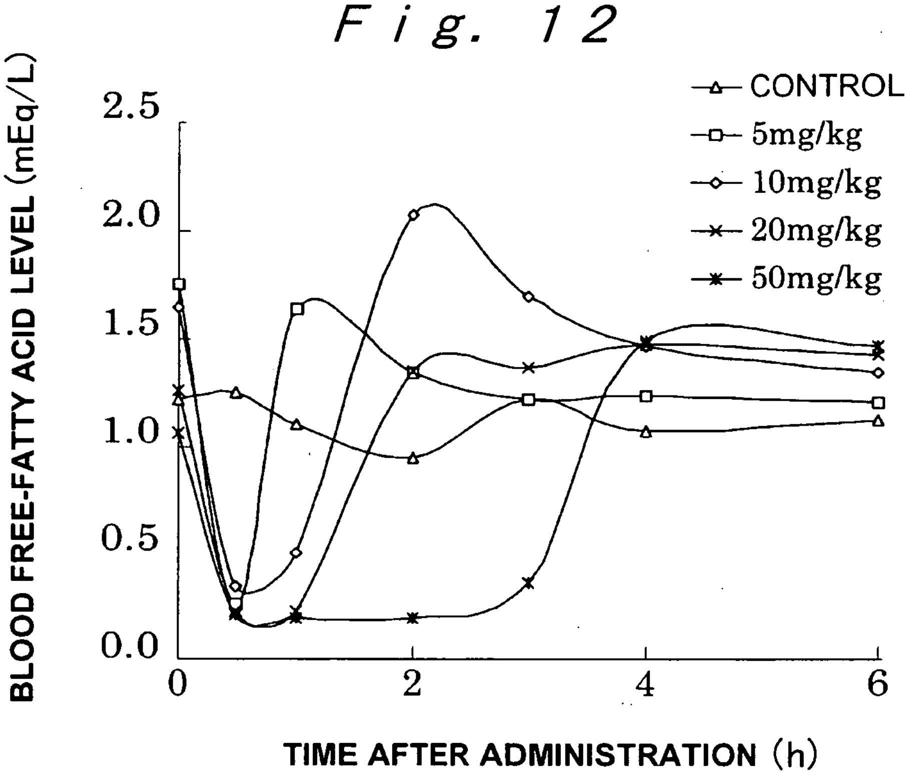
BLOOD FREE-FATTY ACID LEVEL (mEq/L)
2.5
2.0
1.5
1.0
0.5
0.0
0
2
4
6
TIME AFTER ADMINISTRATION (h)
CONTROL
5mg/kg
10mg/kg
20mg/kg
50mg/kg

Fig. 13

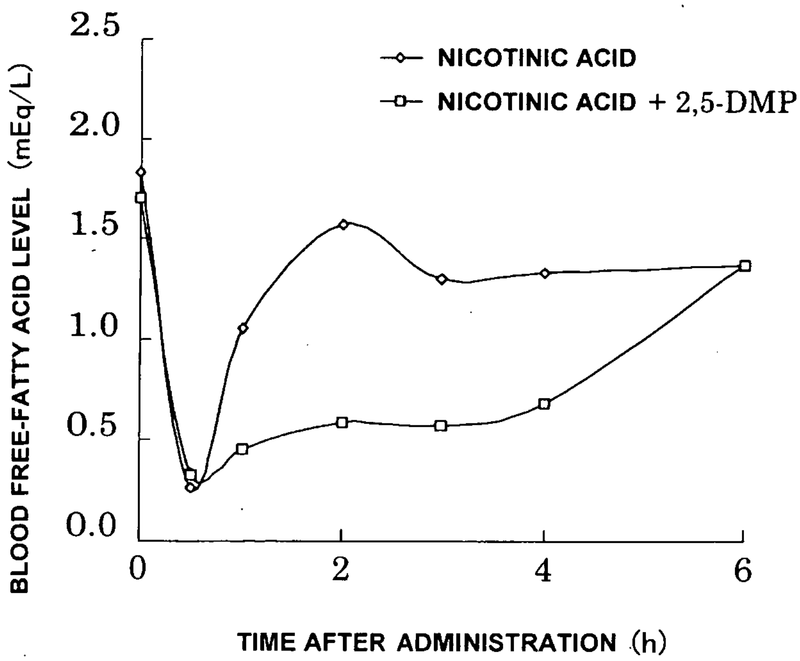
BLOOD FREE-FATTY ACID LEVEL (mEq/L)
2.5
2.0
1.5
1.0
0.5
0.0
0
2
4
6
TIME AFTER ADMINISTRATION (h)
NICOTINIC ACID
NICOTINIC ACID + 2,5-DMP

## INTERNATIONAL SEARCH REPORT

International application No.
PCT/JP2005/018394

A. CLASSIFICATION OF SUBJECT MATTER
***A23F5/18***(2006.01), ***A23F5/04***(2006.01), *A23L1/30*(2006.01), *A61K31/455*(2006.01), *A61K36/18*(2006.01), *A61K36/00*(2006.01), *A61P3/02*(2006.01), *A61P3/06*(2006.01), *A61P3/10*(2006.01)

According to International Patent Classification (IPC) or to both national classification and IPC

B. FIELDS SEARCHED

Minimum documentation searched (classification system followed by classification symbols)
***A23F5/18***(2006.01), ***A23F5/04***(2006.01), *A23L1/30*(2006.01), *A61K31/455*(2006.01), *A61K36/18*(2006.01), *A61K36/00*(2006.01), *A61P3/02*(2006.01), *A61P3/06*(2006.01), *A61P3/10*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
PubMed, JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X | JP 2003-274862 A (UNICAFE INC.), 30 September, 2003 (30.09.03), Full text (Family: none) | 1-48 |
| X | JP 2004-24094 A (UCC Ueshima Coffee Co., Ltd.), 29 January, 2004 (29.01.04), Full text (Family: none) | 1-48 |
| A | H. TAGUCHI, M. SAKAGUCHI, Y. SHIMABAYASHI, 'Trigonelline Content in Coffee Beans and the Thermal Conversion of Trigonelline into Nicotinic Acid during the Roasting of Coffee Beans', Agric.Biol.Chem., 1985, Vol.49, No.12, pages 3467 to 3471 | 1-48 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 December, 2005 (13.12.05) | Date of mailing of the international search report<br>27 December, 2005 (27.12.05) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/018394

C (Continuation). DOCUMENTS CONSIDERED TO BE RELEVANT

| Category* | Citation of document, with indication, where appropriate, of the relevant passages | Relevant to claim No. |
|---|---|---|
| A | Jin ISHII, 'Coffee no Kibun Seibun', Koryo, 1987, No.155, pages 75 to 92 | 1-48 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004289202 A
- JP 2005046258 A
- JP 2005088882 A


### Non-patent literature cited in the description


- *J. Nutr.,* 2004, vol. 134, 2381-2386
- *Agric. Biol. Chem.,* 1985, vol. 49 (12), 3467-3471
- Nutritional and Toxicological Consequences of Food Processing. Plenum Press, 1991, 49-59
- *Eur. J. Med. Chem.,* 1980, vol. 15, 157-163
- *Arch. Int. Med.,* 2004, vol. 164, 697-705
- *Clin. Pharmacol. Ther.,* 1980, vol. 28 (6), 790-795
- *Am. Heart J.,* 2002, vol. 143, 514-8
- *Am. J. Cardiol.,* 2004, vol. 93, 307-12
- *Am. J. Cardiol.,* 2004, vol. 94, 306-11
- *Lancet,* 2002, vol. 360, 1477-1478
- *Lancet,* 2003, vol. 361, 702-704
- *Annals of Internal Medicine,* 2004, vol. 140, 1-8
- *Journal of Internal Medicine,* 2004, vol. 255, 89-95
- *JAMA,* 2004, vol. 291, 1213-1219
- *Anal. Sci.,* 2004, vol. 20, 325-328
- *Arch. Int. Med.,* 2004, vol. 164, 697-705
- Hypertriglyceridemia Handbook. 148-160
- **M. LAVEZZARI et al.** *J. Int. Med. Res.,* 1989, vol. 17, 373-380
- **P. TORNVALL et al.** *J. Int. Med.,* 1991, vol. 230, 415-421
- **M. F. MCCARTY.** A chlorogenic acid-induced increase in GLP-1 production may mediate the impact of heavy coffee consumption on diabetes risk. *Med. Hypotheses,* 2005, vol. 64, 848-853
- **J. SHEARER et al.** Quinides of roasted coffee enhance insulin action in conscious rats. *J. Nutr.,* 2003, vol. 133, 3529-3532
- **M. FRIEDMAN.** Nutritional and Toxicological Consequence of Food Processing. Plenum Press, 1991, 49-59